# EUROPEAN PATENT APPLICATION

(11) **EP 1 348 700 A2**
(43) Date of publication of application: **01.10.2003**
(21) Application number: 03003195.9
(22) Date of filing: 20.02.2003
(51) Int. Cl.: C07D 213/75, C07C 235/74, C07C 245/08, A61K 7/13

(54) **Composition and method for dyeing dye-susceptible material**

(30) Priority: 29.03.2002 US 368665 P; 29.03.2002 US 368661 P; 28.06.2002 US 392577 P
(71) Applicant: Wella Aktiengesellschaft, 64274 Darmstadt (DE)
(72) Inventor: Rozzell, David Dr., Burbank, CA 91506 (US); Allwohn, Jürgen, Dr., 65558 Burgschwalbach (DE); Chassot, Laurent, Dr., 1724 Praroman (CH); Pasquier, Cécile, Dr., 1723 Marly (CH); Sauter, Guido, Dr., 3174 Thörishaus (CH); Buclin-Charrière, Véronique, CH-1638 Morlon (CH)

(57) **Abstract**

Composition for colouring dye-susceptible material (preferably keratinous material), containing at least one pro-dye (especially a glutaramide) and at least one enzyme capable of reacting, cleaving, or modifying the enzymatically-labile functionality in the pro-dye to form a dye substance or a direct dye; as well as a 2-component-kit.

## Description

This invention relates to a composition for colouring dye-susceptible material, containing at least one pro-dye and at least one enzyme capable of reacting, cleaving, or modifying the enzymatically-labile functionality in the pro-dye to form a dye substance or direct dye. This invention further relates to a method for dyeing a dye-susceptible material with a dye substance or a direct dye, in which a dye substance or a direct dye is prepared as a pro-dye, and the pro-dye is converted into a dye substance or a direct dye by an enzyme-catalyzed reaction. The method for dyeing described herein can be used to color any dye-susceptible material, including natural and synthetic textiles and keratinous fibers such as hair.

### Background of the Invention

Methods for dyeing of materials, including natural and synthetic textiles, fibers for carpets, and keratinous fibers such as hair have been widely sought. For dyeing keratin fibers, and in particular the hair, it is known to use oxidation dyes (oxidation bases and couplers) which are compounds that are initially colorless or weakly colored and give rise, by the action of an oxidizing agent, to colored compounds by a process of oxidative condensation or so-called direct dyes, which do not need the action of an oxidation agent.
However, problems are encountered. Some dye substances are unstable towards components in the dyeing formulation (e.g. oxidants or alkaline additives), others are insoluble in the dye formulations. Problems also arise if the dye substance has an insufficient temperature stability to be stored for extended periods of time. In such cases, practical application of such a dye substance is difficult, even if the coloration properties are otherwise highly desirable.

The inventors have now discovered, surprisingly and unexpectedly that a derivative of a dye substance or a direct dye with an enzymatically-labile functionality, referred to herein as a pro-dye, when combined with a suitable enzyme is able to dye dye-susceptible material like keratineous material without the shortcomings of existing dyeing methods. The enzyme cleaves the enzymatically-labile functionality, liberating the dye substance or a direct dye, which then colors the dye-susceptible material. The pro-dyes are very stable towards oxygen or oxidants and alkaline conditions.

### Detailed Description of the Invention

As used herein, "dye-susceptible material" means any tangible object, including but not limited to natural and synthetic fibers, textiles, paper, plastics, polymers, skins, leathers, and the like, including particularly keratinous fibers such as hair. As used herein, "dye substance " means any chemical composition that is capable of dyeing or coloring a dye-susceptible material, including chemical compositions that undergo combination or chemical modification prior to dyeing, including oxydative dye precursors that -alone or in combination with other oxidative dye precursors- are capable of dyeing or coloring a dye-susceptible material, after mixing with an oxidizing agent (e.g. hydrogen peroxide or atmospheric oxygen). As used herein, "direct dye" means a chemical composition that is capable of dyeing or coloring a dye-susceptible material without further chemical modification. As used herein, "pro-dye" means a precursor or derivative of a dye substance or a direct dye with an enzymatically-labile functionality, and which is converted into a dye substance or a direct dye in the presence of a suitable enzyme that can catalyze a reaction with said enzymatically-labile functionality. As used herein, "enzymatically-labile functionality" means a chemical functional group that can be reacted, cleaved, or modified in the presence of a suitable enzyme.

Advantages of the method of this invention include the following:
1) The pro-dye can be constructed so as to have increased stability relative to the stability of the dye, enabling more facile formulation and increased shelf life.
2) Many direct dyes are highly insoluble in dyeing solutions, particularly aqueous dyeing solutions. The pro-dye can be constructed so as to have higher solubility in the dyeing solution, resulting in the ability to achieve higher effective concentrations of the dye, increased rates of dyeing, and deeper and more intense colors.
3) The use of pro-dyes provides the ability to vary the concentrations of the dye over a wider range, giving greater range of colors and hues.
4) Certain dye substances are relatively unstable to air, but when prepared as a suitable pro-dye, greater stability to oxygen can be achieved.
5) Oxidative dye precursors can be prepared as more stable pro-dyes, with enzymatic cleavage to the oxidation-reactive dye precursor used to release the oxidative dye precursor in the presence of the oxidant (e.g. hydrogen peroxide) at the time when dyeing is desired. The benefits include greater stability and ease of formulation.

In the practice of this invention, examples of dye substances and direct dyes that are useful as the basis for the preparation of a pro-dye include nitro benzenes, nitroanilines and various substituted nitroanilines, azo dyes, anthraquinone dyes, phenylenediamines and ring-substituted derivatives, nitrophenols, catechols, aminocatechols, and the like. Pro-dyes are designed so that the enzymatically-labile functionality will, in the presence of a suitable enzyme, cause release or formation of the desired dye substance or direct dye. In a particularly preferred embodiment, the dye substance or direct dye is converted into a pro-dye derivative that, upon enzyme-catalyzed hydrolysis, will release the desired dye substance or direct dye.

It is also possible to prepare and use pro-dyes based on known oxidative dye precursors, e.g. o-, m- or p-diaminobenzes and their derivatives, resorcinol and its derivatives, 4,5-diaminopyrazoles, or m- or p-aminophenols and their derivatives.

Suitable pro-dyes that can serve as precursors for dye substance or direct dye containing one or more amine functional groups include any amide derivatives in which the amide can be hydrolyzed by an amidase, protease, peptidase, or other amide-hydrolyzing enzyme. Suitable pro-dyes include amide derivatives such as acetamides, propionamides, butyramides, benzamides, phenylacetamides, substituted phenylacetamides, succinamides, glutaramides, adipic acid amides, phthalamides, and the like. Other amides derivatives useful as pro-dyes in the practice of this invention include amides formed from amino acids that can be cleaved with a suitable protease, amidase, or peptidase. Amino acids from which pro-dye amides can be formed include both natural amino acids, such as those used as the building blocks of most proteins and non-naturally occurring amino acids. Examples of amino acids useful in the preparation of pro-dye amides with amine-containing dye substances and direct dyes include, but are not limited to leucine (cleaved, for example by leucine aminopeptidase); phenylalanine, tyrosine, or tryptophan (cleaved, for example, by chymostrypsin); lysine, arginine, or ornithine, cleaved, for example, by trypsin); aspartic acid, glutamic acid, or methionine (cleaved, for example by papain); alanine, valine, homoserine, and serine (cleaved, for example by subtlisin); proline and hydroxyproline (cleaved, for example by proline aminopeptidase); and many other examples. Non-naturally occurring amino acids that may be used and cleaved with suitable proteases and amidases include, but are not limited to phenylglycine, p-hydroxy-phenylglycine, 2- aminobutyric acid , 2-aminoadipic acid, and the like.

Suitable pro-dyes that can serve as precursors for dye substance or direct dye containing one or more hydroxy functional groups include any ester derivatives in which the ester can be hydrolyzed by an esterase, lipase, protease, peptidase, amidase, or other ester-hydrolyzing enzyme. Suitable pro-dyes include ester derivatives such as acetates, propionates, butyrates, benzoates, substituted benzoates, phenylacetates, substituted phenylacetates, succinates, glutarates, adipates, phthalates, palmitates, oleates, stearates, glycerates, gluconates, and the like. Ester derivatives of amino acids can also be used. In general, the same or similar enzymes used to hydrolyze amino acid amide derivatives can also be used to hydrolyze amino acid ester derivatives. Thus, phenylalanine, tyrosine, or tryptophan esters can be used and cleaved with, for example, chymostrypsin; lysine, arginine, or ornithine esters can be used and cleaved, for example, by trypsin; aspartic acid, glutamic acid, or methionine esters can be used and cleaved, for example by papain; alanine, valine, homoserine, and serine esters can be used and cleaved, for example by subtlisin; proline and hydroxyproline esters can be used and cleaved, for example by proline aminopeptidase. It will be evident to those skilled in the art that many other pro-dye derivatives with enzyme-cleavable groups and suitable enzymes exist, and that as the enzyme can be used a single enzyme or an enzyme mixture, and such broad application is explicitly contemplated as a part of this invention.

In a preferred embodiment, pro-dyes of oxidative dye precursors are formed, which are more resistant to oxidation than the oxidative dye precursor itself, facilitating the formulation and improving the shelf life of the formulation. As a specific example, the di-glutaramide of p-phenylenediamine can be used as a pro-dye. When dyeing is desired, the di-glutaramide pro-dye is contacted with the enzyme glutaryl acylase, which releases the p-phenylenediamine by hydrolysis of the glutaryl amide bonds. Similarly, the mono-or di-glutaryl derivative of p-aminophenol can be prepared as a pro-dye and hydrolyzed with glutaryl acylase to release the desired p-aminophenol as an oxidative dye precursor. As an alternative, the phenylacetamide derivative of p-phenylene-diamine can be prepared as a pro-dye. When dyeing is desired, contacting the pro-dye with the enzyme penicillin amidase hydrolyzes the amide bond, releasing the oxidative dye precursor for participation in a dyeing step.

A further object of the invention therefore are glutaramide derivatives of formula (I) or their physiologically acceptable salts 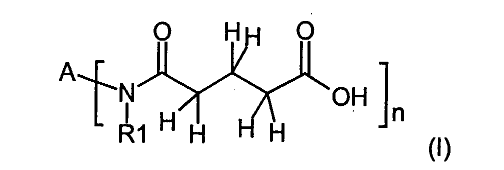 in which
**R1** denotes a hydrogen atom, a C1-C4-alkyl radical or a C1-C4-hydroxyalkyl radical;
**A** denotes the aromatic or heteroaromatic moiety of a mono or diamino dye precursor
and **n** is 1 or 2;
and cosmetic compositions for dyeing keratin fibers, particularly human hairs, containing the above glutaramide derivatives of formula (I).

Examples for suitable compounds of formula (I) are mono-glutaramide or di-glutaramide pro-dyes of 1,4-Diamino-2-methyl-benzene (p-Toluylene diamine), 1,4-Diamino-2,6-dimethyl-benzene, 1,4-Diamino-3,5-diethyl-benzene, 1,4-Diamino-2,5-dimethyl-benzene, 1,4-Diamino-2,3-dimethyl-benzene, 2-Chlor-1,4-diaminobenzene, 1,4-Diamino-2-(thiophen-2-yl)benzene, 1,4-Diamino-2-(thiophen-3-yl)benzene, 1,4-Diamino-2-(pyridin-3-yl)benzene, 2,5-Diaminobiphenyl, 1,4-Diamino-2-methoxymethyl-benzene, 1,4-Diamino-2-aminomethyl-benzene, 1,4-Diamino-2-hydroxymethyl-benzene, 1,4-Diamino-2-(2-hydroxyethoxy)-benzene, 2-(2-(Acetylamino)ethoxy)-1,4-diamino-benzene, 4-Phenylamino-anilin, 4-Dimethylamino-anilin, 4-Diethylamino-anilin, 4-Dipropylamino-anilin, 4-[Ethyl(2-hydroxyethyl)amino]-anilin, 4-[Di(2-hydroxyethyl)amino]-anilin, 4-[Di(2-hydroxyethyl)amino]-2-methyl-anilin, 4-[(2-Methoxyethyl)amino]-anilin, 4-[(3-Hydroxypropyl)amino]-anilin, 4-[(2,3-Dihydroxypropyl)amino]-anilin, 1,4-Diamino-2-(1-hydroxyethyl)benzene, 1,4-Diamino-2-(2-hydroxyethyl)-benzene, 1,4-Diamino-2-(1-methylethyl)-benzene, 1,3-Bis[(4-aminophenyl)(2-hydroxyethyl)amino]-2-propanol, 1,4-Bis[(4-Aminophenyl)amino]-butane, 1,8-Bis(2,5-diamino-phenoxy)-3,6-dioxaoctane, 4-Amino-phenol, 4-Amino-3-methyl-phenol, 4-Amino-3-(hydroxymethyl)-phenol, 4-Amino-3-fluor-phenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxyethyl)amino]methyl-phenol, 4-Amino-2-methyl-phenol, 4-Amino-2-(methoxymethyl)phenol, 4-Amino-2-(2-hydroxyethyl)-phenol, 5-Amino-salicylic acid, 2,5-Diamino-pyridin, 2,4,5,6-Tetraamino-pyrimidin, 2,5,6-Triamino-4-(1H)-pyrimidone, 4,5-Diamino-1-(2-hydroxyethyl)-1H-pyrazol, 4,5-Diamino-1-(1-methylethyl)-1H-pyrazol, 4,5-Diamino-1-[(4-methylphenyl)methyl]-1H-pyrazol, 1-[(4-Chlorphenyl)methyl]-4,5-diamino-1H-pyrazol, 4,5-Diamino-1-methyl-1H-pyrazol, 2-Amino-phenol, 2-Amino-6-methyl-phenol or 2-Amino-5-methyl-phenol.

Preferred compounds of formula (I) are those in which **A** denotes a benzene ring or a pyrazol ring; e.g. 4-(4-amino-phenylcarbamoyl)-butyric acid, 4-[4-(4-carboxy-butyrylamino)-phenylcarbamoyl]-butyric acid, 4-(4-hydroxy-phenylcarbamoyl)-butyric acid, 4-[5-Amino-1-(2-hydroxy-ethyl)-1H-pyrazol-4-ylcarbamoyl]-butyric acid, 4-(2-aminomethyl-4-amino-phenylcarbamoyl)-butyric acid, 4-(2-chloro-4-amino-phenylcarbamoyl)-butyric acid, 4-(2-methyl-4-amino-phenylcarbamoyl)-butyric acid, 4-(2-phenyl-4-amino-phenylcarbamoyl)-butyric acid, 4-(2-pyridyl-4-amino-phenylcarbamoyl)-butyric acid, 4-(2-thienyl-4-amino-phenylcarbamoyl)-butyric acid, 4-(3-2-hydroxyethyl-4-amino-phenylcarbamoyl)-butyric acid, 4-(3-aminomethyl-4-amino-phenylcarbamoyl)-butyric acid, 4-(3-chloro-4-amino-phenylcarbamoyl)-butyric acid, 4-(3-methyl-4-amino-phenylcarbamoyl)-butyric acid, 4-(3-phenyl-4-amino-phenylcarbamoyl)-butyric acid, 4-(3-pyridyl-4-amino-phenylcarbamoyl)-butyric acid, 4-(3-thienyl-4-amino-phenylcarbamoyl)-butyric acid, 4-(2-methyl-4-(4-Carboxy-butyrylamino)-phenylcarbamoyl)-butyric acid, 4-(2-phenyl-4-(4-carboxy-butyrylamino)-phenylcarbamoyl)-butyric acid, 4-(2-pyridyl-4-(4-carboxy-butyrylamino)-phenylcarbamoyl)-butyric acid, 4-(2-thienyl-4-(4-carboxy-butyrylamino)-phenylcarbamoyl)-butyric acid, 4-(3-(2-hydroxyethyl)-4-(4-carboxy-butyrylamino)-phenylcarbamoyl)-butyric acid, 4-(3-aminomethyl-4-(4-carboxy-butyrylamino)-phenylcarbamoyl)-butyric acid, 4-(3-chloro-4-(4-carboxy-butyrylamino)-phenylcarbamoyl)-butyric acid, 4-(3-methyl-4-(4-carboxy-butyrylamino)-phenylcarbamoyl)-butyric acid, 4-(3-phenyl-4-(4-carboxy-butyrylamino)-phenylcarbamoyl)-butyric acid, 4-(3-pyridyl-4-(4-carboxy-butyrylamino)-phenylcarbamoyl)-butyric acid, 4-(3-thienyl-4-(4-carboxy-butyrylamino)-phenylcarbamoyl)-butyric acid, 4-(2-methyl-4-hydroxy-phenylcarbamoyl)-butyric acid, 4-(3-methyl-4-hydroxy-phenylcarbamoyl)-butyric acid, 4-[5-Amino-1-(isopropyl)-1H-pyrazol-4-ylcarbamoyl]-butyric acid, 4-[5-Amino-1-(phenyl)-1H-pyrazol-4-ylcarbamoyl]-butyric acid, 4-[5-Amino-1-(benzyl)-1 H-pyrazol-4-ylcarbamoyl]-butyric acid, 4-[5-(4-Carboxy-butyrylamino)-1-(isopropyl)-1H-pyrazol-4-ylcarbamoyl]-butyric acid, 4-[5-(4-Carboxy-butyrylamino)-1-(phenyl)-1H-pyrazol-4-ylcarbamoyl]-butyric acid and 4-[5-(4-Carboxy-butyrylamino)-1-(benzyl)-1H-pyrazol-4-ylcarbamoyl]-butyric acid.

Particularly preferred compounds of formula (I) are 4-[4-(4-carboxy-butyrylamino)-phenylcarbamoyl]-butyric acid, 4-[5-Amino-1-(2-hydroxy-ethyl)-1H-pyrazol-4-ylcarbamoyl]-butyric acid, 4-(2-methyl-4-amino-phenylcarbamoyl)-butyric acid, 4-[5-Amino-1-(isopropyl)-1H-pyrazol-4-ylcarbamoyl]-butyric acid, 4-(3-(2-hydroxyethyl)-4-(4-carboxy-butyrylamino)-phenylcarbamoyl)-butyric acid.

A further object of the present invention are glutaramide derivatives of direct dyes of formula (la) or their physiologically acceptable salts, 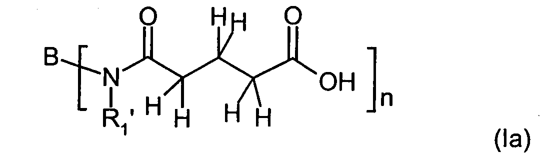 in which
**R1'** denotes hydrogen, a C1-C4-alkyl radical or a C1-C4-hydroxyalkyl radical,
**n** is 1 or 2,
**B** denotes a residue selected from the group consisting of
(i) nitrobenzenes of the formula (II), 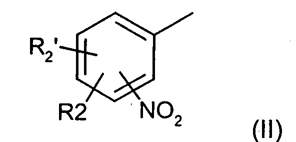 in which R2' denotes hydrogen and R2 denotes hydrogen, a halogen atom, a cyano group, a nitro group, a C1-C4-alkyl radical, a trifluoromethyl radical, a hydroxy group, a C1-C4-alkoxy radical, a C1-C4-hydroxyalkoxy radical, an amino group, a (C1-C4-alkyl)amino radical, a (C1-C4-hydroxy-alkyl)amino radical, a di(C1-C4-alkyl)amino radical, a (C1-C4-alkyl-(C1-C4-hydroxyalkyl)amino radical, a di(C1-C4-hydroxyalkyl)amino radical, a (C1-C4-alkyl)-(C1-C4-hydroxyalkyl)amino radical, an amino-(C1-C4-alkyl) radical, a -(C1-C4-alkyl)-NH-CO-NH2 group, a -COOH group, a -CONH2 group or a N-substituted phenyl-amino group, or R2' and R2 together form a -NH-(C1-C2-alkyl)-NH- bridge or a -NH-(C1-C2-alkyl)-O- bridge;
(ii) nitro-pyridines of the formula (III), 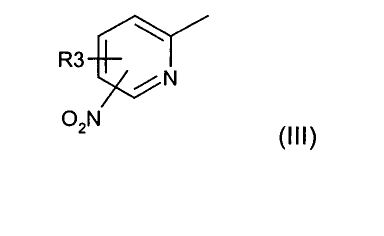 in which R3 denotes hydrogen, an amino group, a C1-C4-alkylamino radical or a (C1-C4-hydroxyalkyl)amino radical;
(iii) anthraquinones of the formula (IV), 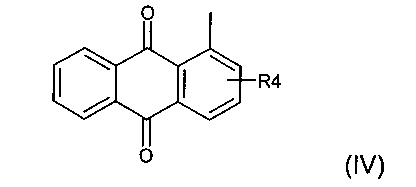 in which R4 denotes hydrogen, an amino group, a C1-C4-alkylamino radical, a (C1-C4-hydroxyalkyl)amino radical, a -NH-(C1-C4-alkyl)-NH2 group, a hydroxy group or a C1-C4-alkoxy radical;
(iv) azo-dyes of the formula (V), 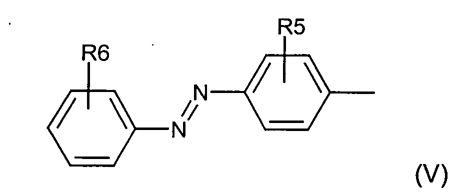 in which R5 denotes hydrogen, an amino group, a nitro group, a C1-C4-alkyl radical or a C1-C4-alkoxy radical, and R6 denotes hydrogen, a C1-C4-alkyl radical, a nitro group, a hydroxy group, a C1-C4-alkylamino radical, a (C1-C4-hydroxyalkyl)amino radical, a di(C1-C4-alkyl)amino radical, a di(C1-C4-hydroxyalkyl)amino radical or a tri(C1-C4-alkyl)-ammonium radical;
(v) azo-dyes of the formula (VI), 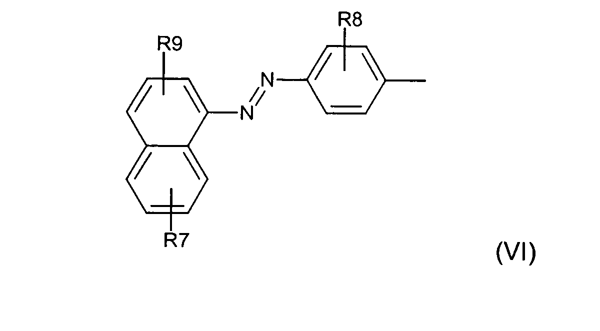 in which R7 and R9 independently of each other denote hydrogen, a C1-C4-alkyl radical, a nitro group, a hydroxy group, a C1-C4-alkylamino radical, a (C1-C4-hydroxyalkyl)amino radical, a di(C1-C4-alkyl)amino radical, a di(C1-C4-hydroxyalkyl)amino radical or a tri(C1-C4-alkyl)-ammonium radical, and R8 denotes hydrogen, an amino group, a nitro group, a C1-C4-alkyl radical or a C1-C4-alkoxy radical;
(vi) azo-dyes of the formula (VII), 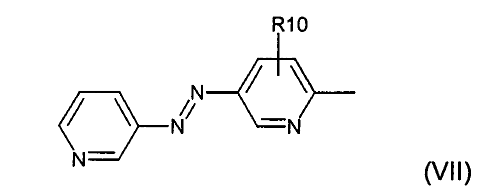 in which R10 denotes hydrogen, an amino group, a nitro group, a C1-C4-alkyl radical or a C1-C4-alkoxy radical; and
(vii) naphthoquinoneimine-dyes of the formula (VIII), 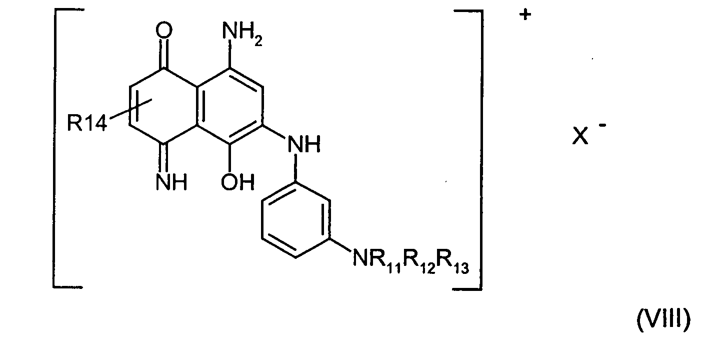 in which R11, R12 and R13 independently of each other denote hydrogen or a C1-C4-alkyl radical; R14 denotes a halogen atom, hydrogen or a C1-C4-alkyl radical; and X⁻ denotes an anion.

According to this invention, amine-containing direct dyes that are useful as the basis for the preparation of a pro-dye according to formula (la) especially include nitroanilines, amino-nitrophenols, amino-nitropyridines, aminoanthraquinones, amino-azodyes and the like.

Examples for suitable pro-dyes of formula (la) are mono-glutaramide or di-glutaramide derivatives of the following direct dyes: 1-Amino-3-methyl-4-[(2-hydroxyethyl)amino]-6-nitrobenzene (HC Violet No. 1), 2-((4-Amino-2-nitrophenyl)amino)-5-dimethylamino-benzoic acid (HC Blue No. 13), 1-Amino-4-[(2-hydroxyethyl)amino]-2-nitrobenzene (HC Red No. 7), 2-Amino-4,6-dinitro-phenol, 1,4-Diamino-2-nitrobenzene (CI76070), 4-Amino-2-nitro-diphenylamine (HC Red No. 1), 1-Amino-4-[di(2-hydroxyethyl)amino]-2-nitrobenzene-hydrochloride (HC Red No. 13), 1-Amino-5-chloro-4-[(2-hydroxyethyl)amino]-2-nitrobenzene, 4-Amino-1-[(2-hydroxyethyl)amino]-2-nitrobenzene (HC Red No. 3), 1-Amino-4-((2,3-dihydroxypropyl)amino)-5-methyl-2-nitrobenzene, 1-Amino-4-(methylamino)-2-nitrobenzene, 4-Amino-2-nitro-1-((prop-2-en-1-yl)amino)-benzene, 4-Amino-3-nitrophenol, 1-Amino-5-chloro-4-[(2,3-dihydroxypropyl)amino]-2-nitrobenzene (HC Red No. 10), 2-[(4-Amino-2-nitrophenyl)amino]-benzoic acid, 2-Amino-6-chloro-4-nitrophenol-hydrochloride, 2,5-diamino-6-nitropyridine, 6-Amino-3-((2-hydroxy-ethyl)amino)-2-nitropyridine, 3-Amino-6-((2-hydroxyethyl)amino)-2-nitro-pyridine, 3-Amino-6-(ethylamino)-2-nitropyridine, 3-Amino-6-(methylamino)-2-nitropyridine, 7-Amino-3,4-dihydro-6-nitro-2H-1,4-benzoxazine (HC Red No. 14) 1,2-Diamino-4-nitrobenzene (CI76020), 1-Amino-2-[(2-hydroxyethyl)-amino]-5-nitrobenzene (HC Yellow No. 5), 2-Amino-3-nitrophenol, 1-Amino-2-methyl-6-nitrobenzene, 1-Amino-4-((2-aminoethyl)amino)-5-methyl-2-nitro-benzene, 1-Amino-4-hydroxy-9,10-anthraquinone (CI60710, Disperse Red 15), 1-[(3-Aminopropyl)amino]-4-methylamino-9,10- anthraquinone (HC Blue No. 8), 1-[(3-Aminopropyl)-amino]-9,10- anthraquinone (HC Red No. 8), 1,4-Diamino-2-methoxy-9,10-anthraquinone (CI62015, Disperse Red No. 11), 1,4-Diamino-9,10-anthraquinone (CI61100, Disperse Violet No. 1), 1-Amino-4-(methylamino)-9,10- anthraquinone (CI61105, Disperse Violet No. 4), Basic Blue 22, 8-Amino-2-bromo-5-hydroxy-4-imino-6-[(3-(trimethylammonio)phenyl)-amino]-1(4H)-naphthalinone-chloride (CI56059; Basic Blue No. 99), Tri(4-amino-3-methylphenyl)carbenium-chloride (CI42520; Basic Violet No. 2), Di(4-aminophenyl)(4-amino-3-methylphenyl)carbenium-chloride (CI42510; Basic Violet No. 14), 1,3-Bis[(2,4-diamino-5-methylphenyl)azo]-3-methylbenzol (CI21010;Basic Brown No. 4), 1-[(4-Amino-phenyl)azo]-7-(trimethylammonio)-2-naphthol-chloride (CI12250; Basic Brown No. 16), 3-[(4-Amino-2,5-dimethoxyphenyl)azo]-N,N,N-trimethyl-benzeneaminium-chloride (CI112605, Basic Orange No. 69), 1-[(4-Amino-2-nitrophenyl)-azo]-7-(trimethylammonio)-2-naphthol-chloride (Basic Brown No. 17), 1-[(4-Amino-3-nitrophenyl)azo]-7-(trimethylammonio)-2-naphthol-chloride (CI12251; Basic Brown No. 17), 3,7-Diamino-2,8-dimethyl-5-phenyl-phenazinium-chloride (CI50240; Basic Red No. 2), 1-[Di(2-hydroxyethyl)-amino]-4-[(4-nitrophenyl)azo]-benzene (Disperse Black No. 9), 4-[(4-Amino-phenyl)azo]-1-[di(2-hydroxyethyl)amino]-3-methylbenzene (HC Yellow No. 7) and 2,6-Diamino-3-((pyridin-3-yl)azo)pyridine.

Particularly preferred compounds of formula (la) are 5-[5-amino-6-nitro-2-(pyridinyl)amino]-5-oxopentanoic acid and 5-[(6-amino-5-(3-pyridinyl-diazenyl)-2-pyridinyl)amino]-5-oxopentanoic acid.

Pro-dye derivatives of reactive dyes can also be prepared. For example, the glutaramide derivative of p-aminobenzaldehyde, or a substituted analog thereof, can be used as a stable pro-dye precursor. The pro-dye is stable and unreactive until contacted with the enzyme glutaryl acylase, whereupon the reactive p-aminobenzaldehyde, or substituted analog thereof, is released.

A further object of the present invention are cosmetic compositions for dyeing keratin fibers, particularly human hairs, containing pro-dyes, especially the above glutaramide derivatives of formula (I) and (la).

The ready-to-use dyeing composition contains from about 0.01 to about 25 percent by weight of said pro-dye, especially of said compound of formula (I) and/or (la); a concentration of from about 0.02 to 4 percent by weight being particularly preferred.

The pro-dyes of formula (I) usually are used in combination with other couplers or developpers. As coupler substance preferably can be used resorcinol, 4-chlororesorcinol, 4,6-dichlororesorcinol, 2-methylresorcinol, 2-amino-4-(2'-hydroxyethyl)-aminoanisole, m-phenylenediamine, 5-amino-2-methylphenol, 2,4-diamino-phenoxyethanol, 4-amino-2-hydroxy-phenoxyethanol, 1-naphthol, 3-aminophenol, 3-amino-2-methylphenol, 4-hydroxy-1,2-methylenedioxy-benzene, 4-amino-1,2-methylenedioxybenzene, 2,4-diamino-5-methyl-phenetole, 4-hydroxyindole and 3,5-diamino-2,6-dimethoxypyridine.

The coupler and developer substances can be contained in the dye compostions according to the invention either alone or as mixtures which each other. The total amount of coupler substances and developer substances in the dye composition according to the invention (relative to the total amount of the dye composition) is from about 0.01 to 28 percent by weight respectively, preferably from about 0.02 to 8.0 by weight and especially from 0.2 to 6.0 percent by weight.

For optimizing the coloring result and achieving specific color effects, additional compounds from the group of direct-acting dyestuffs, for example acid dyes, basic dyes, aromatic nitro dyestuffs, azo dyestuffs, anthraquinone dyestuffs or triphenylmethane dyestuffs may be added, either alone or as a mixture thereof, to the coloring preparation according to the invention. Suitable nitro dyestuffs are, for example, picramic acid, 4-(2',3'-dihydroxy-propyl)-amino-3-nitro-trifluoro-methyl benzene, 4,N-ethyl-N-(2'-hydroxyethyl)-amino-1-(2'-hydroxyethyl)-amino-2-nitrobenzene, 2-chloro-6-ethylamino-4-nitrophenol, 1-hydroxy-2-β-hydroxyethylamino-4,6-dinitrobenzene, 4-(2'-hydroxyethyl)-amino-3-nitro-chlorobenzene, 2-amino-6-chloro-4-nitrophenol and 4-(2'-hydroxyethyl)-amino-3-nitro-methyl benzene.
Suitable azo dyestuffs are, for example, 1-(2'-methoxyphenylazo)-2-hydroxy-7-trimethyl-ammonium-naphthalene (Basic Red 76), 4-(4'-sulfo-1-phenylazo)-1-(4"-sulfophenyl)-3-carboxy-5-hydroxypyrazolon (Acid Yellow 23), 4-amino-4'-bis[2"-hydroxyethyl]-amino-azobenzene (Disperse Black 9) and 1-(4'-aminophenylazo)-2-hydroxy-7-trimethyl-ammonium-naphthalene (Basic Braun 16). Suitable anthraquinone dyestuffs are, for example, 1-methylamino-4-(2'-hydroxyethyl)amino-anthraquinone (Disperse Blue 3), 1-amino-4-hydroxy-anthraquinone (Disperse Red 15), 2-methoxy-1,4-diamino-anthraquinone (Disperse Red 11), 1,4-diamino-anthraquinone (Disperse Violet 1), 1-amino-4-methylamino-anthraquinone (Disperse Violet 4), 1,4-bis(2',3'-dihydroxypropyl)amino-anthraquinone, 1-methylamino-4-(amino-n-propyltrimethylammonium)-anthraquinone (Basic Blue 22), 1,4-bis-(2-hydroxyethyl)amino-5,8-dihydroxy-anthraquinone (Disperse Blue 7) and 1-methylamino-4-aminopropylamino-anthraquinone (HC Blue 8).
Examples for triphenylmethane dyestuffs are [4-[[4-diethylamino]-phenyl][4-(ethylamino)-1-naphthyl]methylene]-2,5-cyclohexadiene-1-ylidene]-N-ethyl-ethaneamine (Basic Blue 7) and 4',4',4"-triamino-3-methyl triphenylcarbenium chloride (Basic Violet 14, Fuchsin AN).

The preferred amount of additional direct-acting dyestuffs to be used is from about 0.01% to about 5% by weight of the total dyeing composition. An especially preferred amount of additional direct-acting dyestuffs to be used is from about 0.1% to about 4% by weight of the total dyeing composition.

The dyeing composition may be prepared in various physical forms. For example, a solution, preferably an aqueous or aqueous alcoholic solution can be used. Alternatively, the dyeing composition may be prepared as a cream, gel, emulsion, powder or solid with a granular consistency. The composition consists of a mixture of the dye components with, optionally, other cosmetic additives usual for such preparations.

Usual cosmetic additives are, for example, solvents, such as water, low aliphatic monohydroxy or polyhydroxy alcohols, their esters and ethers, for example alkanols, particularly with an alkyl chain comprising 1 to 4 carbon atoms, such as ethanol, n-propanol or isopropanol, butanol, isobutanol; bivalent or trivalent alcohols, particularly of the type having 2 to 6 carbon atoms, such as ethylene glycol, propylene glycol, 1,3-propanediol, 1,4-butanediol, 1,5-pentanediol, 1,6-hexanediol, 1,2,6-hexanetriol, glycerine, diethylene glycol, dipropylene glycol, polyalkylene glycols, such as triethylene glycol, polyethylene glycol, tripropylene glycol and polypropylene glycol; low alkyl ethers of multivalent alcohols, such as ethylene glycol monomethyl ether, ethylene glycol monoethyl ether, ethylene glycol monopropyl ether or ethylene glycol monobutyl ether, diethylene glycol monomethyl ether or diethylene glycol mono ethyl ether, triethylene glycol monomethyl ether or triethylene glycol mono ethyl ether; ketones and keto alcohols, especially such with 3 to 7 carbon atoms in their molecules, such as acetone, methyl ethyl ketone, diethyl ketone, methyl isobutyl ketone, methyl phenyl ketone, cyclopentanone, cyclohexanone and diacetone alcohol; ethers such as dibutyl ether, tetrahydrofurane, dioxane or diisopropyl ether; esters such as ethyl formate, methyl formate, methyl acetate, ethyl acetate, propyl acetate, butyl acetate, phenyl acetate, ethylene glycol monoethyl ether acetate or acetic acid hydroxy ethyl ester; amids such as dimethyl formamide, dimethyl acetamide or N-methyl-pyrrolidone; as well as urea, tetramethyl urea and thiodiglycol; moreover, wetting agents or emulsifyers from the group of anionic, cationic, non-ionogenic, amphoteric or zwitterionic surface-active substances, such as fatty aclohol sulfates, alkyl-sulfonates, alkylbenzene sulfonates, alkyltrimethyl ammonium salts, alkylbetaines, α-olefin sulfonates, oxethylated fatty alcohols, oxethylated nonylphenoles, fatty acid alkanolamines, oxethylated fatty acid esters, fatty alcohol polyglycol ether sulfates, alkylpolyglucosides; thickening agents, such as high fatty alcohols, starch, cellulose derivatives, vaseline, paraffin oil, fatty acids and other fatty components in emulsified form, water-soluble polymeric thickening agents, for instance natural rubbers, guar gum, xanthan gum, carob flour, pectine, dextrane, agar-agar, amylose, amylopectine, dextrine, clays or synthetic hydrocolloides, such as polyvinyl alcohol; also conditioning agents, such as lanolin derivatives, cholesterol, pantothenic acid, water-soluble cationic polymers, protein derivatives, provitamins, vitamins, plant extracts, sugar and betaine; auxiliary agents, such as electrolytes, antioxidants, fatty amides, sequestrants, film-forming agents and preservatives.

The aforementioned compounds are used in the amounts usual for such purposes, for example, wetting agents and emulsifying agents in concentrations of about 0.5 to 30 % by weight, thickening agents of about 0.1 to 25 % by weight, conditioning agents in concentrations of about 0.1 to 5.0 % by weight.

In a further embodiment, this invention is directed toward dyeing compositions for coloring dye-susceptible materials, including but not limited to natural and synthetic fibers, textiles, paper, plastics, polymers, skins, leathers, and the like. In a particularly preferred embodiment, compositions in accord with this invention can be used to dye keratinous fibers such as hair.

Compositions in accord with this invention contain a pro-dye -especially a compound of formula (I) or (la)- and an enzyme capable of reacting, cleaving, or modifying the enzymatically-labile functionality in the pro-dye to form a dye substance or direct dye.

The content of pro-dye in these compositions preferably varies from 0.01 to 20 percent by weight, relative to the amount of dyestuff in the pro-dye.

The aforesaid pro-dye and enzyme components of the dyeing composition of this invention may be prepared, for example, as a solution, emulsion, a suspension or similar. In a preferred embodiment, the solution is an aqueous or aqueous alcoholic solution. In other embodiments, the composition may be prepared as a cream, gel, emulsion, powder or as a solid of granular consistency. The composition consists of, at a minimum, a mixture of the pro-dye and enzyme.

As already described, for optimizing the coloring result and achieving specific color effects, further compounds from the group of direct-acting dyestuffs, for example acid dyes, basic dyes, aromatic nitro dyestuffs, azo dyestuffs, anthraquinone dyestuffs or triphenylmethane dyestuffs may be added, either alone or as a mixture thereof, to the coloring composition according to the invention. Compositions useful in the practice of this invention may therefore contain additional compounds in addition to the pro-dye and suitable enzyme. Suitable nitro dyestuffs that may be added are, for example, picramic acid, 4-(2',3'-dihydroxypropyl)-amino-3-nitro-trifluoro-methyl benzene, 4,N-ethyl-N-(2'-hydroxyethyl)-amino-1-(2'-hydroxyethyl)-amino-2-nitrobenzene, 2-chloro-6-ethylamino-4-nitrophenol, 1-hydroxy-2-β-hydroxyethylamino-4,6-dinitrobenzene, 4-(2'-hydroxyethyl)-amino-3-nitro-chlorobenzene, 2-amino-6-chloro-4-nitrophenol and 4-(2'-hydroxyethyl)-amino-3-nitro-methyl benzene. Suitable azo dyestuffs are, for example, 1-(2'-methoxyphenylazo)-2-hydroxy-7-trimethyl-ammonium-naphthalene (Basic Red 76), 4-(4'-sulfo-1-phenylazo)-1-(4"-sulfophenyl)-3-carboxy-5-hydroxypyrazolon (Acid Yellow 23), 4-amino-4'-bis[2"-hydroxyethyl]-amino-azobenzene (Disperse Black 9) and 1-(4'-amino-phenylazo)-2-hydroxy-7-trimethyl-ammonium-naphthalene (Basic Brown 16). Suitable anthraquinone dyestuffs are, for example, 1-methylamino-4-(2'-hydroxyethyl)amino-anthraquinone (Disperse Blue 3), 1-amino-4-hydroxy-anthraquinone (Disperse Red 15), 2-methoxy-1,4-diamino-anthraquinone (Disperse Red 11), 1,4-diamino-anthraquinone (Disperse Violet 1), 1-amino-4-methylamino-anthraquinone (Disperse Violet 4), 1,4-bis(2',3'-dihydroxy-propyl)amino-anthraquinone, 1-methylamino-4-(amino-n-propyltrimethyl-ammonium)-anthraquinone (Basic Blue 22), 1,4-bis-(2-hydroxyethyl)amino-5,8-dihydroxy-anthraquinone (Disperse Blue 7) and 1-methylamino-4-aminopropylamino-anthraquinone (HC Blue 8).
Examples for triphenylmethane dyestuffs are [4-[[4-diethylamino]-phenyl][4-(ethylamino)-1-naphthyl]methylene]-2,5-cyclohexadiene-1-ylidene]-N-ethyl-ethaneamine (Basic Blue 7) and 4',4',4"-triamino-3-methyl triphenylcarbenium chloride (Basic Violet 14, Fuchsin AN).

It is also possible to add known oxidative dye precursors, e.g. o-diaminobenzes, m-diaminobenzes or p-diaminobenzes and their derivatives, resorcinol and its derivatives, 4,5-diaminopyrazoles or m-aminophenols or p-aminophenols and their derivatives.

The preferred amount of additional direct-acting dyestuffs and/or oxidative dye precursors to be used is from about 0.01% to about 5% by weight. An especially preferred amount of additional direct-acting dyestuffs and/or oxidative dye precursors to be used is from about 0.1 % to about 4% by weight.

The additional components of the dyeing composition may be, for example, a solution, preferably an aqueous or aqueous alcoholic solution, or may be of cream, gel, emulsion, powder or granular consistency.

Optionally, the composition may contain additional components or cosmetic additives usual for such preparations. In the case of a composition for the coloration of hair, for example, usual cosmetic additives such as water, low aliphatic monohydroxy or polyhydroxy alcohols, their esters and ethers, for example alkanols, particularly with an alkyl chain comprising 1 to 4 carbon atoms, such as ethanol, n-propanol or isopropanol, butanol, isobutanol; bivalent or trivalent alcohols, particularly of the type having 2 to 6 carbon atoms, such as ethylene glycol, propylene glycol, 1,3-propanediol, 1,4-butanediol, 1,5-pentanediol, 1,6-hexanediol, 1,2,6-hexanetriol, glycerine, diethylene glycol, dipropylene glycol, polyalkylene glycols, such as triethylene glycol, polyethylene glycol, tripropylene glycol and polypropylene glycol; low alkyl ethers of multivalent alcohols, such as ethylene glycol monomethyl ether, ethylene glycol monoethyl ether, ethylene glycol monopropyl ether or ethylene glycol monobutyl ether, diethylene glycol monomethyl ether or diethylene glycol mono ethyl ether, triethylene glycol monomethyl ether or triethylene glycol mono ethyl ether; ketones and keto alcohols, especially such with 3 to 7 carbon atoms in their molecules, such as acetone, methyl ethyl ketone, diethyl ketone, methyl isobutyl ketone, methyl phenyl ketone, cyclopentanone, cyclohexanone and diacetone alcohol; ethers such as dibutyl ether, tetrahydrofurane, dioxane or diisopropyl ether; esters such as ethyl formate, methyl formate, methyl acetate, ethyl acetate, propyl acetate, butyl acetate, phenyl acetate, ethylene glycol monoethyl ether acetate or acetic acid hydroxy ethyl ester; amids such as dimethyl formamide, dimethyl acetamide or N-methyl-pyrrolidone; as well as urea, tetramethyl urea and thiodiglycol; moreover, wetting agents or emulsifyers from the group of anionic, cationic, non-ionogenic, amphoteric or zwitterionic surface-active substances, such as fatty aclohol sulfates, alkyl-sulfonates, alkylbenzene sulfonates, alkyltrimethyl ammonium salts, alkylbetaines, α-olefin sulfonates, oxethylated fatty alcohols, oxethylated nonylphenoles, fatty acid alkanolamines, oxethylated fatty acid esters, fatty alcohol polyglycol ether sulfates, alkylpolyglucosides; thickening agents, such as high fatty alcohols, starch, cellulose derivatives, vaseline, paraffin oil, fatty acids and other fatty components in emulsified form, water-soluble polymeric thickening agents, for instance natural rubbers, guar gum, xanthan gum, carob flour, pectine, dextrane, agar-agar, amylose, amylopectine, dextrine, clays or synthetic hydrocolloides, such as polyvinyl alcohol; also conditioning agents, such as lanolin derivatives, cholesterol, pantothenic acid, water-soluble cationic polymers, protein derivatives, provitamins, vitamins, plant extracts, sugar and betaine; auxiliary agents, such as electrolytes, antioxidants, fatty amides, sequestrants, film-forming agents and preservatives.

The aforementioned compounds are used in the amounts usual for such purposes, for example, wetting agents and emulsifying agents in concentrations of about 0.5% to about 30% by weight, thickening agents of from about 0.1% to about 25% by weight, conditioning agents in concentrations of form about 0.1% to about 5.0 % by weight.

In a particularly preferred embodiment, the pro-dye and enzyme are prepared as separate components (A) and (B), and the two components are combined just before use to form the ready-to-apply dyeing composition.

In another embodiment, the pro-dye and enzyme are combined and stored in an aqueous solution at a pH at which the enzyme and pro-dye are stable but at which the enzyme-catalyzed reaction does not take place. The pH is then adjusted into the range at which the enzyme is catalytically active at the time dyeing is desired.

The dyeing compositions according to this invention may be applied in various ways.
If the pro-dye is used without oxidizing agents, the components (A) and (B) may be mixed prior to the application and be spread on the material to be colored either immediately or after an incubation time of 15 to 45 minutes. The mixture is left on the material to be colored, such as hair, at about 15 to 50 °C, preferably 25 to 45 °C, for a time period of from about 1 minute to about 75 minutes, and more preferably of from about 25 minutes to about 40 minutes. The material is then rinsed with water and dried. In the case of hair coloring, after rinsing, the hair may be washed with a shampoo and/or subsequently be rinsed with a weak organic acid, such as citric acid, glycolic acid, lactic acid, malic acid, ascorbic acid or tartaric acid.

If pro-dyes of oxidative dye precursors are used or an additional lightening of the material (e.g. hair) is intended, it is also possible to mix the components (A) and (B) with an oxidizing agent (e.g. a 1 to 12 percent hydrogen peroxide or its addition complexes containing aqueous solution) prior to use and to apply the mixture afterwards. In this case the mixture is left on the material to be colored, such as hair, at about 15 to 50 °C, preferably 25 to 45 °C, for a time period of 10 to 45 minutes, preferably of 25 to 40 minutes. The dyed material is then rinsed with water and dried. In the case of hair coloring, after rinsing, the hair may be washed with shampoo and/or subsequently be rinsed with a weak organic acid, such as citric acid, glycolic acid, lactic acid, malic acid, ascorbic acid or tartaric acid.

The dyeing preparation is applied at a pH of from about 4 to about 10.5, more preferably of from about 6.0 to about 10, most preferably of from about 7.0 to about 9.5.

It is also possible, if desired, to add to the component (A) of the dyeing composition a thio compound having a reducing action, such as β-mercaptoethanol, sodium thioglycolate or dithiothreitol. In the case of hair dyeing, such thiol compounds are particularly useful.

The dyeing composition/dyeing method according to the present invention achieves (especially on hair) color results with excellent fastness properties, especially with regard to light, shampooing and friction. Depending on the type and concentration of the compounds of the general formula (I), a variety of different color shades can be obtained with the coloring preparation according to this invention. The high intensity and purity of the colors obtained with the preparation according to the invention are particularly noteworthy. In addition, the described coloring preparation also permits coloring grey and chemically undamaged hair without problems and obtaining a very good covering effect. The color results thus achieved are, irrespective of different hair structures, uniform and highly reproducible.

The invention is now described in more detail with the following examples, which are for illustrative purposes and are not intended to limit the scope of the invention.

### Examples

### A. Preparation of pro-dyes

### Example 1: Preparation of di-glutaramide of p-phenylenediamine

To a mixture of 0.9 g (5 mmol) of p-phenylenediamine dihydrochloride and 2.0 ml of triethylamine in 30 ml of chloroform, a solution of 1.14 g (10 mmol) of glutaric anhydride in 20 ml of chloroform was added dropwise at 0°C. The mixture was allowed to warm up to room temperature and stirred for 3 h, and then was poured into 50 ml of 5% aqueous HCI. A white precipitate was formed, and filtration yielded a colorless solid. The colorless solid so obtained was washed with water and dichloromethane. After drying, the product di-glutaramide of p-phenylenediamine weighed 1.37 grams (91% yield).

### Example 2: Preparation of di-phenylacetamide of p-phenylenediamine

To a mixture of 0.9 g (5 mmol) of p-phenylenediamine dihydrochloride and 2.0 ml of triethylamine in 30 ml of chloroform, a solution of 1.45 g (10 mmol) of phenylacetic chloride in 20 ml of chloroform was added dropwise at 0°C. The mixture was allowed to warm up to room temperature, and then was poured into 50 ml of 5% aqueous HCI. The organic layer was separated, and the aqueous layer was extracted twice with 30 ml of chloroform. The combined organic extracts were washed twice with H₂O and dried over anhydrous Na₂SO₄. The solvent was removed to give the product di-phenylacetamide of p-phenylenediamine as a colorless solid (1.47 g, 90% yield).

### Example 3: Preparation of glutaramide pro-dye for p-aminobenzaldehyde

To a solution of 0.61 grams (5.0 mmol) of p-aminobenzaldehyde in 10 ml of chloroform containing two drops of triethylamine, a solution of 0.57 g (5 mmol) of glutaric anhydride in 10 ml of chloroform was added dropwise at 0°C. The mixture was allowed to warm up to room temperature and then stirred for 3 hours, and then the reaction mixture was poured into 30 ml of 5% aqueous HCI. The organic layer was separated in a separatory funnel, and the aqueous layer was extracted with 20 ml of chloroform. The combined organic extracts were washed twice with H₂O and dried over anhydrous Na₂SO₄. The solvent was removed by evaporation to give the product glutaramide of p-aminobenzaldehyde as a colorless solid (0.9 g, 82% yield).

### Example 4: Preparation of the phenylacetamide of p-aminobenzylalcohol

To a solution of 0.61 grams (5.0 mmol) of aminobenzylalcohol in 10 ml of chloroform and two drops of triethylamine, a solution of 0.72 g (5 mmol) of phenylacetic chloride in 10 ml of chloroform was added dropwise at 0°C. The mixture was allowed to warm up to room temperature slowly, and then was poured into 30 ml of 5% aqueous HCI. The organic layer was separated, and the aqueous layer was extracted with 20 ml of chloroform. The combined organic extracts were washed twice with H₂O and dried over anhydrous Na₂SO₄. The solvent was removed to give a pale yellow solid, which was recrystallized in toluene to give the phenylacetamide of aminobenzylalcokol as a colorless solid (1.02 g, 88% yield).

### Example 5: Preparation of glutaramide pro-dye for p-aminophenol

To a solution of 0.55 grams (5.0 mmol) of p-aminophenol in 10 ml of chloroform containing two drops of triethylamine, a solution of 0.57 g (5 mmol) of glutaric anhydride in 10 ml of chloroform is added dropwise at 0°C. The mixture is allowed to warm up to room temperature and then stirred for 3 hours, and then the reaction mixture is poured into 30 ml of 5% aqueous HCI. The organic layer is separated in a separatory funnel, and the aqueous layer is extracted with 20 ml of chloroform. The combined organic extracts are washed twice with H₂O and dried over anhydrous Na₂SO₄. The solvent is removed by evaporation to give the product glutaramide as a colorless solid (0.85 g, 82% yield).

### Example 6: Preparation of phenylacetamide pro-dye for p-aminophenol

To a solution of 0.55 grams (5.0 mmol) of p-aminophenol in 10 ml of chloroform and two drops of triethylamine, a solution of 0.72 g (5 mmol) of phenylacetic chloride in 10 ml of chloroform was added dropwise at 0°C. The mixture was allowed to warm up to room temperature slowly, and then was poured into 30 ml of 5% aqueous HCI. The organic layer was separated, and the aqueous layer was extracted with 20 ml of chloroform. The combined organic extracts were washed twice with H₂O and dried over anhydrous Na₂SO₄. The solvent was removed to give a pale yellow solid, which was recrystallized in toluene to give the phenylacetamide as a colorless solid.

### Example 7: Preparation of glutaramide pro-dye for o-phenylenediamine

To a mixture of 0.9 g (5 mmol) of o-phenylenediamine dihydrochloride and 2.0 ml of triethylamine in 30 ml of chloroform, a solution of 1.14 g (10 mmol) of glutaric anhydride in 20 ml of chloroform is added dropwise at 0°C. The mixture is allowed to warm up to room temperature and stirred for 3 h, and then is poured into 50 ml of 5% aqueous HCI. A white precipitate is formed, and filtration yielded a colorless solid. The colorless solid so obtained is washed with water and dichloromethane. After drying, the product is obtained as a colorless solid.

### Example 8: Preparation of phenylacetamide pro-dye for o-phenylenediamine

To a mixture of 0.9 g (5 mmol) of o-phenylenediamine dihydrochloride and 2.0 ml of triethylamine in 30 ml of chloroform, a solution of 1.45 g (10 mmol) of phenylacetic chloride in 20 ml of chloroform is added dropwise at 0°C. The mixture is allowed to warm up to room temperature, and then is poured into 50 ml of 5% aqueous HCI. The organic layer was separated, and the aqueous layer is extracted twice with 30 ml of chloroform. The combined organic extracts are washed twice with H₂O and dried over anhydrous Na₂SO₄. The solvent is removed to give the product phenylacetamide (mixture of the mono- and di-phenylacetamide) of p-phenylenediamine as a colorless solid.

### Example 9: Preparation of L-phenylalanine amide pro-dye for p-phenylenediamine

To a mixture of 0.9 g (5 mmol) of p-phenylenediamine dihydrochloride and 2.0 ml of triethylamine in 30 ml of chloroform, a solution of 2 g (5.5 mmol) of N-t-BOC-L-phenylalanine N-hydroxysuccinimidyl ester (Sigma Chemical Company, St. Louis, MO; product number B1880) in 20 ml of chloroform is added dropwise at 0°C. The mixture is allowed to warm up to room temperature, and then is poured into 50 ml of 5% aqueous HCI. After stirring the aqueous mixture for 4 hours at room temperature, the organic layer is separated and discarded, and the aqueous layer is extracted twice with 30 ml of chloroform. The aqueous layer is evaporated to dryness under vacuum to leave the product L-phenylalanine amide of p-phenylenediamine hydrochloride salt as a residue.

### Example 10: Preparation of L-phenylalanine amide pro-dye for p-aminobenzyl-alcohol

To a mixture of 0.55 g (5 mmol) of p-aminobenzylalcohol and 2.0 ml of triethylamine in 30 ml of chloroform, a solution of 2 g (5.5 mmol) of N-t-BOC-L-phenylalanine N-hydroxysuccinimidyl ester (Sigma Chemical Company, St. Louis, MO; product number B1880) in 20 ml of chloroform is added dropwise at 0°C. The mixture is allowed to warm up to room temperature, and then is poured into 50 ml of 5% aqueous HCI. After stirring the aqueous mixture for 4 hours at room temperature, the organic layer is separated and discarded, and the aqueous layer is extracted twice with 30 ml of chloroform. The aqueous layer is evaporated to dryness under vacuum to leave the product L-phenylalanine amide of p-aminobenzylalcohol hydrochloride salt as a residue.

### Example 11: Preparation of 5-[5-amino-6-nitro-2-(pyridinyl)amino]-5-oxopentanoic acid (=glutaramide of 2,5-diamino-6-nitropyridine)

To a mixture of 3.08g (20 mmol) of 2,5-diamino-6-nitropyridine and 0.36g (3mmol) of N,N-dimethylamino-pyridine in 120 ml of tetrahydrofurane, 5.7 g (50 mmol) of glutaric anhydride were added at room temperature. The mixture was stirred for 3 hours at room temperature and then refluxed for additional 3 hours. The mixture was then filtered, the solvent was evaporated and the crude product was dissolved in ethylacetate and extracted three times with a 1N sodium hydroxide solution. The combined aqueous extracts were acidified with conc. HCI at 0 °C. A precipitate was formed, and filtration yielded an orange solid. The solid so obtained was washed with water. After drying 4,33 g (81% yield) of 5-[5-amino-6-nitro-2-pyridinyl)amino]-5-oxopentanoic acid were obtained.

### Example 12: Preparation of 5-[(6-amino-5-(3-pyridinyldiazenyl)-2-pyridinyl)amino]-5-oxopentanoic acid (=glutaramide of 2,6-diamino-3-((pyridin-3-yl)azo)pyridine)

To a mixture of 3.21g (15 mmol) of 2,6-diamino-3-((pyridin-3-yl)azo)pyridine and 0.27g (2.2 mmol) of N,N-dimethylamino-pyridine in 100 ml of tetrahydrofurane, 4.27 g (37.5 mmol) of glutaric anhydride was added at room temperature. The mixture was refluxed for 16 hours. The mixture was then filtered, the solvent was evaporated and the crude product was dissolved in ethylacetate and extracted three times with a 1N sodium hydroxide solution. The combined aqueous extracts were acidified with conc. HCI at 0 °C. A precipitate was formed, and filtration yielded an orange solid. The solid so obtained was washed with water. After drying the product was recristallised in methanol/water mixture and 1.9 g (40 % yield) of 5-[(6-amino-5-(3-pyridinyldiazenyl)-2-pyridinyl)amino]-5-oxopentanoic acid were obtained.

### Examples 13-59: Preparation of glutaramides of alternative amine-containing direct dyes to produce pro-dyes

Procedures analogous to those used in Example 11 or Example 12 can be repeated with minor modifications to produce mono-glutaramide or di-glutaramide pro-dyes from the following 47 amine-containing direct dyes:
**13.** 1-Amino-3-methyl-4-[(2-hydroxyethyl)amino]-6-nitrobenzene (HC Violet No. 1),
**14**. 2-((4-Amino-2-nitrophenyl)amino)-5-dimethylamino-benzoic acid (HC Blue No. 13),
**15**. 1-Amino-4-[(2-hydroxyethyl)amino]-2-nitrobenzene (HC Red No. 7),
**16**. 2-Amino-4,6-dinitro-phenol,
**17.** 1,4-Diamino-2-nitrobenzene (CI76070),
**18.** 4-Amino-2-nitro-diphenylamine (HC Red No. 1),
**19.** 1-Amino-4-[di(2-hydroxyethyl)amino]-2-nitrobenzene-hydrochloride (HC Red No. 13),
**20.** 1-Amino-5-chloro-4-[(2-hydroxyethyl)amino]-2-nitrobenzene,
**21.** 4-Amino-1-[(2-hydroxyethyl)amino]-2-nitrobenzene (HC Red No. 3),
**22.** 1-Amino-4-((2,3-dihydroxypropyl)amino)-5-methyl-2-nitrobenzene,
**23.** 1-Amino-4-(methylamino)-2-nitrobenzene,
**24.** 4-Amino-2-nitro-1-((prop-2-en-1-yl)amino)-benzene,
25. 4-Amino-3-nitrophenol,
**26.** 1-Amino-5-chloro-4-[(2,3-dihydroxypropyl)amino]-2-nitrobenzene (HC Red No. 10),
**27.** 2-((4-Amino-2-nitrophenyl)amino)-benzoic acid
**28.** 2-Amino-6-chloro-4-nitrophenol-hydrochloride,
**29.** 6-Amino-3-((2-hydroxyethyl)amino)-2-nitropyridine,
**30**. 3-Amino-6-((2-hydroxyethyl)amino)-2-nitropyridine,
**31.** 3-Amino-6-(ethylamino)-2-nitropyridine,
**32.** 3-Amino-6-(methylamino)-2-nitropyridine,
**33.** 7-Amino-3,4-dihydro-6-nitro-2H-1,4-benzoxazine (HC Red No. 14)
**34.** 1,2-Diamino-4-nitrobenzene (CI76020),
**35.** 1-Amino-2-[(2-hydroxyethyl)amino]-5-nitrobenzene (HC Yellow No. 5),
**36.** 2-Amino-3-nitrophenol,
**37.** 1-Amino-2-methyl-6-nitrobenzene,
**38.** 1-Amino-4-((2-aminoethyl)amino)-5-methyl-2-nitrobenzene,
**39.** 1-Amino-4-hydroxy-9,10-anthraquinone (CI60710, Disperse Red 15),
**40.** 1-[(3-Aminopropyl)amino]-4-methylamino-9,10- anthraquinone (HC Blue No. 8),
**41.** 1-[(3-Aminopropyl)amino]-9,10- anthraquinone (HC Red No. 8),
**42.** 1,4-Diamino-2-methoxy-9,10- anthraquinone (CI62015, Disperse Red No. 11),
**43.** 1,4-Diamino-9,10- anthraquinone (CI61100, Disperse Violet No. 1),
**44.** 1-Amino-4-(methylamino)-9,10- anthraquinone (CI61105, Disperse Violet No. 4, Solvent Violet No. 12)
**45.** 8-Amino-2-bromo-5-hydroxy-4-imino-6-[(3-(trimethylammonio)phenyl)-amino]-1(4H)-naphthalinone-chloride (CI56059; Basic Blue No. 99),
**46.** Tri(4-amino-3-methylphenyl)carbenium-chloride (CI42520; Basic Violet No. 2),
**47**. Di(4-aminophenyl)(4-amino-3-methylphenyl)carbenium-chloride (CI42510; Basic Violet No. 14),
**48**. 1,3- Bis[(2,4-diamino-5-methylphenyl)azo]-3-methylbenzol (CI21010;Basic Brown No. 4),
**49.** 1-[(4-Aminophenyl)azo]-7-(trimethylammonio)-2-naphthol-chloride (CI12250; Basic Brown No. 16),
**50.** 3-[(4-Amino-2,5-dimethoxyphenyl)azo]-N,N,N-trimethylbenzeneaminium-chloride (CI112605, Basic Orange No. 69),
**51.** 1-[(4-Amino-2-nitrophenyl)azo]-7-(trimethylammonio)-2-naphthol-chlorid (CI12251:1; Basic Red No. 118),
**52.** 1-[(4-Amino-3-nitrophenyl)azo]-7-(trimethylammonio)-2-naphthol-chlorid (CI12251; Basic Brown No. 17),
**53.** 3,7-Diamino-2,8-dimethyl-5-phenylphenazinium-chloride (CI50240; Basic Red No. 2),
**54.** 1-[Di(2-hydroxyethyl)amino]-4-[(4-nitrophenyl)azo]-benzene (Disperse Black No. 9),
**55.** 4-[(4-Aminophenyl)azo]-1-[di(2-hydroxyethyl)amino]-3-methylbenzene (HC Yellow No. 7).
**56.** 1-(Methylamino)-4-((3-(trimethylammonium)propyl)amino)-9,10-anthracendion-chlorid (Basic Blue No. 22),
**57.** 1-(Methylamino)-4-((3-(trimethylammonium)propyl)amino)-9,10-anthracendion-methylsulfat,
**58.** 4-((2-Hydroxyethyl)methylamino)-1-(methylamino)-2-nitrobenzol,
**59.** 4-[(2-Hydroxyethyl)amino]-3-nitrophenol,

### Examples 60-114: Preparation of glutaramides of alternative amine-containing oxidative dyes to produce pro-dyes for use in oxidative dyeing conditions

Procedures analogous to those used in Example 1 or Example 5 can be repeated with minor modifications to produce mono-glutaramide or di-glutaramide pro-dyes from the following 55 amine-containing oxidative dyes:
**60.** 1,4-Diamino-2-methyl-benzol (p-Toluylendiamin),
**61.** 1,4-Diamino-2,6-dimethyl-benzol,
**62.** 1,4-Diamino-3,5-diethyl-benzol,
**63**. 1,4-Diamino-2,5-dimethyl-benzol,
**64.** 1,4-Diamino-2,3-dimethyl-benzol,
**65.** 2-Chlor-1,4-diaminobenzol,
**66.** 1,4-Diamino-2-(thiophen-2-yl)benzol,
**67.** 1,4-Diamino-2-(thiophen-3-yl)benzol,
**68**. 1,4-Diamino-2-(pyridin-3-yl)benzol,
**69**. 2,5-Diaminobiphenyl,
**70.** 1,4-Diamino-2-methoxymethyl-benzol,
**71**. 1,4-Diamino-2-aminomethyl-benzol,
**72**. 1,4-Diamino-2-hydroxymethyl-benzol,
**73**. 1,4-Diamino-2-(2-hydroxyethoxy)-benzol,
**74.** 2-(2-(Acetylamino)ethoxy)-1,4-diamino-benzol,
**75.** 4-Phenylamino-anilin,
**76.** 4-Dimethylamino-anilin,
**77**. 4-Diethylamino-anilin,
**78.** 4-Dipropylamino-anilin,
**79**. 4-[Ethyl(2-hydroxyethyl)amino]-anilin,
**80**. 4-[Di(2-hydroxyethyl)amino]-anilin,
**81**. 4-[Di(2-hydroxyethyl)amino]-2-methyl-anilin,
**82.** 4-[(2-Methoxyethyl)amino]-anilin,
**83.** 4-[(3-Hydroxypropyl)amino]-anilin,
**84.** 4-[(2,3-Dihydroxypropyl)amino]-anilin,
**85.** 1,4-Diamino-2-(1-hydroxyethyl)-benzol,
**86.** 1,4-Diamino-2-(2-hydroxyethyl)-benzol,
**87.** 1,4-Diamino-2-(1-methylethyl)-benzol,
**88.** 1,3-Bis[(4-aminophenyl)(2-hydroxyethyl)amino]-2-propanol,
**89.** 1,4-Bis[(4-Aminophenyl)amino]-butan,
**90.** 1,8-Bis(2,5-diaminophenoxy)-3,6-dioxaoctan,
**91.** 4-Amino-phenol,
**92**. 4-Amino-3-methyl-phenol,
**93**. 4-Amino-3-(hydroxymethyl)-phenol,
**94.** 4-Amino-3-fluor-phenol,
**95.** 4-Methylamino-phenol,
**96**. 4-Amino-2-(aminomethyl)-phenol,
**97.** 4-Amino-2-(hydroxymethyl)-phenol,
**98.** 4-Amino-2-fluor-phenol,
**99.** 4-Amino-2-[(2-hydroxyethyl)-amino]methyl-phenol,
**100.** 4-Amino-2-methyl-phenol,
**101**. 4-Amino-2-(methoxymethyl)-phenol,
**102.** 4-Amino-2-(2-hydroxyethyl)-phenol,
**103.** 5-Amino-salicylsäure,
**104.** 2,5-Diamino-pyridin,
**105.** 2,4,5,6-Tetraamino-pyrimidin,
**106.** 2,5,6-Triamino-4-(1H)-pyrimidon,
**107.** 4,5-Diamino-1-(2-hydroxyethyl)-1H-pyrazol,
**108.** 4,5-Diamino-1-(1-methylethyl)-1H-pyrazol,
**109.** 4,5-Diamino-1-[(4-methylphenyl)methyl]-1H-pyrazol,
**110.** 1 -[(4-Chlorphenyl)methyl]-4,5-diamino-1H-pyrazol,
**111.** 4,5-Diamino-1-methyl-1 H-pyrazol,
**112.** 2-Amino-phenol,
**113.** 2-Amino-6-methyl-phenol,
**114.** 2-Amino-5-methyl-phenol

### Example 115: Preparation of L-lysine amide of p-phenylenediamine

To a mixture of 0.9 g (5 mmol) of p-phenylenediamine dihydrochloride and 2.0 ml of triethylamine in 30 ml of chloroform, a solution of 2.4 g (5.5 mmol) of N,N-alpha,epsilon-di-t-BOC-L-lysine N-hydroxysuccinimidyl ester (Sigma Chemical Company, St. Louis, MO; product number B7019) in 20 ml of chloroform is added dropwise at 0°C. The mixture is allowed to warm up to room temperature, and then is poured into 50 ml of 5% aqueous HCI. After stirring the aqueous mixture for 4 hours at room temperature, the organic layer is separated and discarded, and the aqueous layer is extracted twice with 30 ml of chloroform. The aqueous layer is evaporated to dryness under vacuum to leave the product L-lysine amide of p-phenylenediamine hydrochloride salt as a residue.

### Example 116: Preparation of L-lysine amide of p-aminobenzylalcohol

To a mixture of 0.55 g (5 mmol) of p-aminobenzylalcohol and 2.0 ml of triethylamine in 30 ml of chloroform, a solution of 2.4 g (5.5 mmol) of N,N-alpha,epsilon-di-t-BOC-L-lysine N-hydroxysuccinimidyl ester (Sigma Chemical Company, St. Louis, MO; product number B7019) in 20 ml of chloroform is added dropwise at 0°C. The mixture is allowed to warm up to room temperature, and then is poured into 50 ml of 5% aqueous HCI. After stirring the aqueous mixture for 4 hours at room temperature, the organic layer is separated and discarded, and the aqueous layer is extracted twice with 30 ml of chloroform. The aqueous layer is evaporated to dryness under vacuum to leave the product L-lysine amide of p-aminobenzylalcohol hydrochloride salt as a residue.

### Examples 117-154: Preparation of L-lysine amides of alternative amine-containing direct dyes to produce pro-dyes

Procedures analogous to those used in Example 115 or Example 116 can be repeated with minor modifications to produce L-lysine amide pro-dyes from the following 38 amine-containing direct dyes:
**117.** 1-Amino-4-[(2-hydroxyethyl)amino]-2-nitrobenzol (HC Red No. 7),
**118.** 2-Amino-4,6-dinitro-phenol,
**119.** 1,4-Diamino-2-nitrobenzol (CI76070),
**120.** 4-Amino-2-nitro-diphenylamin (HC Red No. 1),
**121.** 1-Amino-4-[di(2-hydroxyethyl)amino]-2-nitrobenzol-hydrochlorid (HC Red No. 13),
**122.** 1-Amino-5-chlor-4-[(2-hydroxyethyl)amino]-2-nitrobenzol,
**123.** 4-Amino-1-[(2-hydroxyethyl)amino]-2-nitrobenzol (HC Red No. 3),
**124.** 4-((2-Hydroxyethyl)methylamino)-1-(methylamino)-2-nitrobenzol,
**125.** 1-Amino-4-((2,3-dihydroxypropyl)amino)-5-methyl-2-nitrobenzol,
**126.** 1 -Amino-4-(methylamino)-2-nitrobenzol,
**127.** 4-Amino-2-nitro-1-((prop-2-en-1-yl)amino)-benzol,
**128.** 4-Amino-3-nitrophenol,
**129.** 4-[(2-Hydroxyethyl)amino]-3-nitrophenol,
**130.** 1-Amino-5-chlor-4-[(2,3-dihydroxypropyl)amino]-2-nitrobenzol (HC Red No. 10),
**131.** 2-Amino-6-chlor-4-nitrophenol,
**132.** 6-Amino-3-((2-hydroxyethyl)amino)-2-nitropyridin,
**133.** 3-Amino-6-((2-hydroxyethyl)amino)-2-nitropyridin,
**134.** 3-Amino-6-(ethylamino)-2-nitropyridin,
**135.** 3-Amino-6-(methylamino)-2-nitropyridin,
**136.** 7-Amino-3,4-dihydro-6-nitro-2H-1,4-benzoxazin (HC Red No. 14)
**137.** 1,2-Diamino-4-nitrobenzol (CI76020),
**138.** 1-Amino-2-[(2-hydroxyethyl)amino]-5-nitrobenzol (HC Yellow No. 5),
**139.** 2-Amino-3-nitrophenol,
**140.** 1-Amino-2-methyl-6-nitrobenzol,
**141.** 1-Amino-4-((2-aminoethyl)amino)-5-methyl-2-nitrobenzol,
**142.** 1-Amino-4-hydroxy-9,10-anthrachinon (CI60710, Disperse Red 15),
**143.** 1-[(3-Aminopropyl)amino]-4-methylamino-9,10-anthrachinon (HC Blue No. 8),
**144.** 1-[(3-Aminopropyl)amino]-9,10-anthrachinon (HC Red No. 8),
**145.** 1,4-Diamino-2-methoxy-9,10-anthrachinon (CI62015, Disperse Red No. 11, Solvent Violet No. 26),
**146.** 1,4-Diamino-9,10-anthrachinon (CI61100, Disperse Violet No. 1),
**147.** 1-Amino-4-(methylamino)-9,10-anthrachinon (CI61105, Disperse Violet No. 4, Solvent Violet No. 12).
**148.** 8-Amino-2-brom-5-hydroxy-4-imino-6-[(3-(trimethylammonio)phenyl)-amino]-1(4H)-naphthalinon-chlorid (CI56059; Basic Blue No. 99),
**149.** Di(4-aminophenyl)(4-amino-3-methylphenyl)carbenium-chlorid (CI42510; Basic Violet No. 14),
**150.** 1 -[(4-Aminophenyl)azo]-7-(trimethylammonio)-2-naphthol-chlorid (CI12250; Basic Brown No. 16),
**151.** 3-[(4-Amino-2,5-dimethoxyphenyl)azo]-N,N,N-trimethylbenzolaminium-chlorid (CI112605, Basic Orange No. 69),
**152.** 1-[(4-Amino-2-nitrophenyl)azo]-7-(trimethylammonio)-2-naphthol-chlorid (CI12251:1, Basic Red No. 118),
**153.** 1-[(4-Amino-3-nitrophenyl)azo]-7-(trimethylammonio)-2-naphthol-chlorid (CI12251; Basic Brown No. 17),
**154.** 3,7-Diamino-2,8-dimethyl-5-phenylphenazinium-chlorid (CI50240; Basic Red No. 2),

### Examples 155-209: Preparation of L-lysine amides of alternative amine-containing oxidative dyes to produce pro-dyes for use in oxidative dyeing conditions

Procedures analogous to those used in Example 115 or Example 116 can be repeated with minor modifications to produce L-lysine amide pro-dyes from the following 55 amine-containing oxidative dyes:
**155.** 1,4-Diamino-2-methyl-benzol (p-Toluylendiamin),
**156.** 1,4-Diamino-2,6-dimethyl-benzol,
**157.** 1,4-Diamino-3,5-diethyl-benzol,
**158.** 1,4-Diamino-2,5-dimethyl-benzol,
**159.** 1,4-Diamino-2,3-dimethyl-benzol,
**160.** 2-Chlor-1,4-diaminobenzol,
**161.** 1,4-Diamino-2-(thiophen-2-yl)benzol,
**162.** 1,4-Diamino-2-(thiophen-3-yl)benzol,
**163.** 1,4-Diamino-2-(pyridin-3-yl)benzol,
**164.** 2,5-Diaminobiphenyl,
**165.** 1,4-Diamino-2-methoxymethyl-benzol,
**166.** 1,4-Diamino-2-aminomethyl-benzol,
**167.** 1,4-Diamino-2-hydroxymethyl-benzol,
**168.** 1,4-Diamino-2-(2-hydroxyethoxy)-benzol,
**169.** 2-(2-(Acetylamino)ethoxy)-1,4-diamino-benzol,
**170.** 4-Phenylamino-anilin,
**171.** 4-Dimethylamino-anilin,
**172.** 4-Diethylamino-anilin,
**173.** 4-Dipropylamino-anilin,
**174.** 4-[Ethyl(2-hydroxyethyl)amino]-anilin,
**175.** 4-[Di(2-hydroxyethyl)amino]-anilin,
**176.** 4-[Di(2-hydroxyethyl)amino]-2-methyl-anilin,
**177.** 4-[(2-Methoxyethyl)amino]-anilin,
**178.** 4-[(3-Hydroxypropyl)amino]-anilin,
**179.** 4-[(2,3-Dihydroxypropyl)amino]-anilin,
**180.** 1,4-Diamino-2-(1-hydroxyethyl)-benzol,
**181.** 1,4-Diamino-2-(2-hydroxyethyl)-benzol,
**182.** 1,4-Diamino-2-(1-methylethyl)-benzol,
**183.** 1,3-Bis[(4-aminophenyl)(2-hydroxyethyl)amino]-2-propanol,
**184.** 1,4-Bis[(4-Aminophenyl)amino]-butan,
**185.** 1,8-Bis(2,5-diaminophenoxy)-3,6-dioxaoctan,
**186.** 4-Amino-phenol,
**187.** 4-Amino-3-methyl-phenol,
**188.** 4-Amino-3-(hydroxymethyl)-phenol,
**189.** 4-Amino-3-fluor-phenol,
**190.** 4-Methylamino-phenol,
**191.** 4-Amino-2-(aminomethyl)-phenol,
**192.** 4-Amino-2-(hydroxymethyl)-phenol,
**193.** 4-Amino-2-fluor-phenol,
**194.** 4-Amino-2-[(2-hydroxyethyl)-amino]methyl-phenol,
**195.** 4-Amino-2-methyl-phenol,
**196.** 4-Amino-2-(methoxymethyl)-phenol,
**197.** 4-Amino-2-(2-hydroxyethyl)-phenol,
**198.** 5-Amino-salicylsäure,
**199.** 2,5-Diamino-pyridin,
**200.** 2,4,5,6-Tetraamino-pyrimidin,
**201.** 2,5,6-Triamino-4-(1 H)-pyrimidon,
**202.** 4,5-Diamino-1-(2-hydroxyethyl)-1 H-pyrazol,
**203.** 4,5-Diamino-1-(1-methylethyl)-1H-pyrazol,
**204.** 4,5-Diamino-1-[(4-methylphenyl)methyl]-1H-pyrazol,
**205.** 1-[(4-Chlorphenyl)methyl]-4,5-diamino-1H-pyrazol,
**206.** 4,5-Diamino-1-methyl-1H-pyrazol,
**207**. 2-Amino-phenol,
**208.** 2-Amino-6-methyl-phenol,
**209.** 2-Amino-5-methyl-phenol.

### B. Enzymatic generation of a dye substance or direct-dye from a pro-dye

### Example 210: Enzymatic generation of oxidative dye precursor p-phenylenediamine from a pro-dye

A solution of the di-glutaramide of p-phenylenediamine was prepared at a concentration of 100 mM in aqueous 200 mM potassium phosphate buffer, and the pH was adjusted to 7.5 by the addition of 6 M sodium hydroxide solution. The pH-adjusted di-glutaramide solution was diluted 1:10 (final concnetration of 10 mM) into a 200 mM potassium phosphate buffer solution, pH 7.5, containing a suspension of immobilized glutaryl acylase (BioCatalytics, Inc., Pasadena, CA USA; Product Number 1464213) added to achieve a catalytic activity of 104 units per milliliter. As a negative control, an identical reaction mixture was prepared except that the immobilized glutaryl acylase was replaced with penicillin amidase (BioCatalytics, Inc., Pasadena, CA USA; Product Number 1290959) added to achieve a catalytic activity of 400 units per milliliter. The reaction mixtures were mixed gently and the progress of the reaction was followed by thin layer chromatography (silica gel plates, eluent of methanol:methylene chloride 1:10; visualization by UV light). Complete conversion of the di-glutaramide of p-phenylenediamine to phenylenediamine was achieved within 1 hour. In the case of the control reaction with penicillin amidase, no conversion of the di-glutaramide of p-phenylenediamine was detected over a 24 hour period.

### Example 211: Alternative enzymatic generation of oxidative dye precursor p-phenylenediamine from a pro-dye

A suspension of the di-phenylacetamide of p-phenylenediamine was prepared by the addition of 3.44 milligrams per milliliter in aqueous 200 mM potassium phosphate buffer, pH 7.5. Penicillin amidase (BioCatalytics, Inc., Pasadena, CA USA; Product Number 1290959) was added to achieve a final concentration of 200 units per milliliter. As a negative control, an identical reaction mixture was prepared except that the penicillin amidase was replaced with immobilized glutaryl acylase (BioCatalytics, Inc., Pasadena, CA USA; Product Number 1464213) added to achieve a catalytic activity of 104 units per milliliter. The reaction mixtures were mixed gently and the progress of the reaction was followed by thin layer chromatography (silica gel plates, eluent of methanol:methylene chloride 1:10; visualization by UV light). Complete conversion of the di-phenylacetamide of p-phenylenediamine to phenylenediamine was achieved within 1 hour. In the case of the control reaction with glutaryl acylase, no conversion of the di-phenylacetamide of p-phenylenediamine was detected over a 24 hour period.

### Example 212: Enzymatic generation of oxidative dye precursor p-phenylenediamine from a pro-dye in the presence of a co-solvent

A suspension of the di-phenylacetamide of p-phenylenediamine was prepared by the addition of 3.44 milligrams per milliliter in aqueous 200 mM potassium phosphate buffer, pH 7.5 containing 20% v/v dimethylsulfoxide. Penicillin amidase (BioCatalytics, Inc., Pasadena, CA USA; Product Number 1290959) was added to achieve a final concentration of 200 units per milliliter. As a negative control, an identical reaction mixture was prepared except that the penicillin amidase was replaced with immobilized glutaryl acylase (BioCatalytics, Inc., Pasadena, CA USA; Product Number 1464213) added to achieve a catalytic activity of 104 units per milliliter. The reaction mixtures were mixed gently and the progress of the reaction was followed by thin layer chromatography (silica gel plates, eluent of methanol:methylene chloride 1:10; visualization by UV light). Complete conversion of the di-phenylacetamide of p-phenylenediamine to phenylenediamine was achieved within 24 hours. In the case of the control reaction with glutaryl acylase, no conversion of the di-phenylacetamide of p-phenylenediamine was detected over a 24 hour period.

### Example 213: Alternative enzymatic generation of oxidative dye precursor p-phenylenediamine from a mono-phenylacetamide pro-dye

A suspension of the mono-phenylacetamide of p-phenylenediamine was prepared by the addition of 2.6 milligrams per milliliter in aqueous 200 mM potassium phosphate buffer, pH 7.5. Penicillin amidase (BioCatalytics, Inc., Pasadena, CA USA; Product Number 1290959) was added to achieve a final concentration of 200 units per milliliter. As a negative control, an identical reaction mixture was prepared except that the penicillin amidase was replaced with immobilized glutaryl acylase (BioCatalytics, Inc., Pasadena, CA USA; Product Number 1464213) added to achieve a catalytic activity of 104 units per milliliter. The reaction mixtures were mixed gently and the progress of the reaction was followed by thin layer chromatography (silica gel plates, eluent of methanol:methylene chloride 1:10; visualization by UV light). Complete conversion of the mono-phenylacetamide of p-phenylenediamine to phenylenediamine was achieved within 1 hour. In the case of the control reaction with glutaryl acylase, no conversion of the mono-phenylacetamide of p-phenylenediamine was detected over a 24 hour period.

### Example 214: Enzymatic generation of oxidative dye precursor p-phenylenediamine from a mono-phenylacetamide pro-dye in the presence of a co-solvent

A suspension of the mono-phenylacetamide of p-phenylenediamine was prepared by the addition of 2.6 milligrams per milliliter in aqueous 200 mM potassium phosphate buffer, pH 7.5 containing 20% v/v dimethylsulfoxide. Penicillin amidase (BioCatalytics, Inc., Pasadena, CA USA; Product Number 1290959) was added to achieve a final concentration of 200 units per milliliter. As a negative control, an identical reaction mixture was prepared except that the penicillin amidase was replaced with immobilized glutaryl acylase (BioCatalytics, Inc., Pasadena, CA USA; Product Number 1464213) added to achieve a catalytic activity of 104 units per milliliter. The reaction mixtures were mixed gently and the progress of the reaction was followed by thin layer chromatography (silica gel plates, eluent of methanol:methylene chloride 1:10; visualization by UV light). Complete conversion of the mono-phenylacetamide of p-phenylenediamine to phenylenediamine was achieved within 1 hour. In the case of the control reaction with glutaryl acylase, no conversion of the mono-phenylacetamide of p-phenylenediamine was detected over a 24 hour period.

### Example 215: Enzymatic generation of oxidative dye precursor p-aminobenzyl-alcohol from a phenylacetamide pro-dye

A suspension of the phenylacetamide of p-aminobenzylalcohol was prepared by the addition of 2.4 milligrams per milliliter in aqueous 200 mM potassium phosphate buffer, pH 7:5. Penicillin amidase (BioCatalytics, Inc., Pasadena, CA USA; Product Number 1290959) was added to achieve a final concentration of 200 units per milliliter. As a negative control, an identical reaction mixture was prepared except that the penicillin amidase was replaced with immobilized glutaryl acylase (BioCatalytics, Inc., Pasadena, CA USA; Product Number 1464213) added to achieve a catalytic activity of 104 units per milliliter. The reaction mixtures were mixed gently and the progress of the reaction was followed by thin layer chromatography (silica gel plates, eluent of methanol:methylene chloride 1:10; visualization by UV light). Complete conversion of the phenylacetamide of p-aminobenzylalcohol to form p-aminobenzylalcohol was achieved within 1 hour. In the case of the control reaction with glutaryl acylase, no conversion of the phenylacetamide of p-aminobenzylalcohol was detected over a 24 hour period.

### Example 216: Enzymatic generation of oxidative dye precursor p-aminobenzyl-alcohol from a phenylacetamide pro-dye in the presence of a co-solvent

A suspension of the phenylacetamide of p-aminobenzylalcohol was prepared by the addition of 2.4 milligrams per milliliter in aqueous 200 mM potassium phosphate buffer, pH 7.5 containing 20% v/v dimethylsulfoxide. Penicillin amidase (BioCatalytics, Inc., Pasadena, CA USA; Product Number 1290959) was added to achieve a final concentration of 200 units per milliliter. As a negative control, an identical reaction mixture was prepared except that the penicillin amidase was replaced with immobilized glutaryl acylase (BioCatalytics, Inc., Pasadena, CA USA; Product Number 1464213) added to achieve a catalytic activity of 104 units per milliliter. The reaction mixtures were mixed gently and the progress of the reaction was followed by thin layer chromatography (silica gel plates, eluent of methanol:methylene chloride 1:10; visualization by UV light). Complete conversion of the phenylacetamide of p-aminobenzylalcohol to p-aminobenzylalcohol was achieved within 1 hour. In the case of the control reaction with glutaryl acylase, no conversion of the phenylacetamide of p-aminobenzylalcohol was detected over a 24 hour period.

### Example 217: Enzymatic generation of oxidative dye precursor p-aminobenzyl-alcohol from a pro-dye

A solution of the glutaramide of p- aminobenzylalcohol was prepared at a concentration of 100 mM in aqueous 200 mM potassium phosphate buffer, and the pH was adjusted to 7.5 by the addition of 6 M sodium hydroxide solution. The pH-adjusted glutaramide solution was diluted 1:10 (final concentration of 10 mM) into a 200 mM potassium phosphate buffer solution, pH 7.5, containing a suspension of immobilized glutaryl acylase (BioCatalytics, Inc., Pasadena, CA USA; Product Number 1464213) added to achieve a catalytic activity of 104 units per milliliter. As a negative control, an identical reaction mixture was prepared except that the immobilized glutaryl acylase was replaced with penicillin amidase (BioCatalytics, Inc., Pasadena, CA USA; Product Number 1290959) added to achieve a catalytic activity of 400 units per milliliter. The reaction mixtures were mixed gently and the progress of the reaction was followed by thin layer chromatography (silica gel plates, eluent of methanol:methylene chloride 1:10; visualization by UV light). Complete conversion of the glutaramide of p- aminobenzylalcohol to p- aminobenzylalcohol was achieved within 1 hour. In the case of the control reaction with penicillin amidase, no conversion of the glutaramide of p-aminobenzylalcohol was detected over a 24 hour period.

### Example 218: Enzymatic generation of oxidative dye precursor p-phenylenediamine from the L-phenylalanine amide pro-dye of p-phenylenediamine

A solution of the L-phenylalanine amide of p-phenylenediamine is prepared at a concentration of 5 milligrams per milliliter in aqueous 200 mM potassium phosphate buffer, pH 8. Chymotrypsin (Sigma Chemical Company, St. Louis, MO USA; Product Number C4129) was added to achieve a final concentration of 200 units per milliliter. As a negative chymotrypsin is replaced with penicillin amidase (BioCatalytics, Inc., Pasadena, CA USA; Product Number 1290959) added to achieve a catalytic activity of 200 units per milliliter. The reaction mixtures are mixed gently and the progress of the reaction is followed by thin layer chromatography (silica gel plates, eluent of methanol:methylene chloride 1:10; visualization by UV light). Complete conversion of the L-phenylalanine amide of p-phenylenediamine to p-phenylenediamine is achieved within 1 hour. In the case of the control reaction with penicillin amidase, no conversion of the L-phenylalanine amide of p-phenylenediamine is detected over a 12 hour period.

### Example 219: Enzymatic generation of oxidative dye precursor p-phenylene-diamine from the L-alanine amide pro-dye of p-phenylenediamine

A solution of the L-alanine amide of p-phenylenediamine is prepared at a concentration of 5 milligrams per milliliter in aqueous 200 mM potassium phosphate buffer, pH 8. Subtilisin (Sigma Chemical Company, St. Louis, MO USA; Product Number P5380) was added to achieve a final concentration of 200 units per milliliter. As a negative subtilisin is replaced with penicillin amidase (BioCatalytics, Inc., Pasadena, CA USA; Product Number 1290959) added to achieve a catalytic activity of 200 units per milliliter. The reaction mixtures are mixed gently and the progress of the reaction is followed by thin layer chromatography (silica gel plates, eluent of methanol:methylene chloride 1:10; visualization by UV light). Complete conversion of the L-phenylalanine amide of p-phenylenediamine to p-phenylenediamine is achieved within 1 hour. In the case of the control reaction with penicillin amidase, no conversion of the L-alanine amide of p-phenylenediamine is detected over a 12 hour period.

### Example 220: Enzymatic generation of oxidative dye precursor p-phenylene-diamine from the L-aspartic acid amide pro-dye of p-phenylenediamine

A solution of the L- aspartic acid β-amide of p-phenylenediamine is prepared at a concentration of 10 milligrams per milliliter in aqueous 200 mM potassium phosphate buffer, pH 8. Papain (Sigma Chemical Company, St. Louis, MO USA; Product Number P3125) was added to achieve a final concentration of 200 units per milliliter. As a negative subtilisin is replaced with penicillin amidase (BioCatalytics, Inc., Pasadena, CA USA; Product Number 1290959) added to achieve a catalytic activity of 200 units per milliliter. The reaction mixtures are mixed gently and the progress of the reaction is followed by thin layer chromatography (silica gel plates, eluent of methanol:methylene chloride 1:10; visualization by UV light). Complete conversion of the L- aspartic acid β-amide of p-phenylenediamine to p-phenylenediamine is achieved within 1 hour. In the case of the control reaction with penicillin amidase, no conversion of the L- aspartic acid β-amide of p-phenylenediamine is detected over a 12 hour period.

### Example 221: Enzymatic generation of oxidative dye precursor p-phenylenediamine from the L-tyrosine amide pro-dye of p-phenylenediamine

A solution of the L- tyrosine amide of p-phenylenediamine is prepared at a concentration of 5 milligrams per milliliter in aqueous 200 mM potassium phosphate buffer, pH 8. Chymotrypsin (Sigma Chemical Company, St. Louis, MO USA; Product Number C4129) was added to achieve a final concentration of 200 units per milliliter. As a negative chymotrypsin is replaced with penicillin amidase (BioCatalytics, Inc., Pasadena, CA USA; Product Number 1290959) added to achieve a catalytic activity of 200 units per milliliter. The reaction mixtures are mixed gently and the progress of the reaction is followed by thin layer chromatography (silica gel plates, eluent of methanol:methylene chloride 1:10; visualization by UV light). Complete conversion of the L- tyrosine amide of p-phenylenediamine to p-phenylenediamine is achieved within 1 hour. In the case of the control reaction with penicillin amidase, no conversion of the L- tyrosine amide of p-phenylenediamine is detected over a 12 hour period.

### Example 222: Enzymatic generation of oxidative dye precursor p- aminobenzylalcohol from the L-phenylalanine amide pro-dye of p-aminobenzyl-alcohol

A solution of the L-phenylalanine amide of p- aminobenzylalcohol is prepared at a concentration of 5 milligrams per milliliter in aqueous 200 mM potassium phosphate buffer, pH 8. Chymotrypsin (Sigma Chemical Company, St. Louis, MO USA; Product Number C4129) was added to achieve a final concentration of 200 units per milliliter. As a negative chymotrypsin is replaced with penicillin amidase (BioCatalytics, Inc., Pasadena, CA USA; Product Number 1290959) added to achieve a catalytic activity of 200 units per milliliter. The reaction mixtures are mixed gently and the progress of the reaction is followed by thin layer chromatography (silica gel plates, eluent of methanol:methylene chloride 1:10; visualization by UV light). Complete conversion of the L-phenylalanine amide of p- aminobenzylalcohol to p- aminobenzylalcohol is achieved within 1 hour. In the case of the aminobenzylalcohol reaction with penicillin amidase, no conversion of the L-phenylalanine amide of p- aminobenzylalcohol is detected over a 12 hour period.

### Example 223: Enzymatic generation of oxidative dye precursor p- aminobenzylalcohol from the L-alanine amide pro-dye of p-aminobenzylalcohol

A solution of the L-alanine amide of p- aminobenzylalcohol is prepared at a concentration of 5 milligrams per milliliter in aqueous 200 mM potassium phosphate buffer, pH 8. Subtilisin (Sigma Chemical Company, St. Louis, MO USA; Product Number P5380) was added to achieve a final concentration of 200 units per milliliter. As a negative subtilisin is replaced with penicillin amidase (BioCatalytics, Inc., Pasadena, CA USA; Product Number 1290959) added to achieve a catalytic activity of 200 units per milliliter. The reaction mixtures are mixed gently and the progress of the reaction is followed by thin layer chromatography (silica gel plates, eluent of methanol:methylene chloride 1:10; visualization by UV light). Complete conversion of the L-phenylalanine amide of p- aminobenzylalcohol to p- aminobenzylalcohol is achieved within 1 hour. In the case of the control reaction with penicillin amidase, no conversion of the L-alanine amide of p- aminobenzylalcohol is detected over a 12 hour period.

### Example 224: Enzymatic generation of oxidative dye precursor p-aminobenzylalcohol from the L-aspartic acid amide pro-dye of p-aminobenzylalcohol

A solution of the L- aspartic acid β-amide of p- aminobenzylalcohol is prepared at a concentration of 10 milligrams per milliliter in aqueous 200 mM potassium phosphate buffer, pH 8. Papain (Sigma Chemical Company, St. Louis, MO USA; Product Number P3125) was added to achieve a final concentration of 200 units per milliliter. As a negative subtilisin is replaced with penicillin amidase (BioCatalytics, Inc., Pasadena, CA USA; Product Number 1290959) added to achieve a catalytic activity of 200 units per milliliter. The reaction mixtures are mixed gently and the progress of the reaction is followed by thin layer chromatography (silica gel plates, eluent of methanol:methylene chloride 1:10; visualization by UV light). Complete conversion of the L- aspartic acid β-amide of p- aminobenzylalcohol to p- aminobenzylalcohol is achieved within 1 hour. In the case of the control reaction with penicillin amidase, no conversion of the L- aspartic acid β-amide of p- aminobenzylalcohol is detected over a 12 hour period.

### Example 225: Enzymatic generation of oxidative dye precursor p- aminobenzylalcohol from the L-tyrosine amide pro-dye of p- aminobenzylalcohol

A solution of the L- tyrosine amide of p- aminobenzylalcohol is prepared at a concentration of 5 milligrams per milliliter in aqueous 200 mM potassium phosphate buffer, pH 8. Chymotrypsin (Sigma Chemical Company, St. Louis, MO USA; Product Number C4129) was added to achieve a final concentration of 200 units per milliliter. As a negative chymotrypsin is replaced with penicillin amidase (BioCatalytics, Inc., Pasadena, CA USA; Product Number 1290959) added to achieve a catalytic activity of 200 units per milliliter. The reaction mixtures are mixed gently and the progress of the reaction is followed by thin layer chromatography (silica gel plates, eluent of methanol:methylene chloride 1:10; visualization by UV light). Complete conversion of the L- tyrosine amide of p- aminobenzylalcohol to p- aminobenzylalcohol is achieved within 1 hour. In the case of the control reaction with penicillin amidase, no conversion of the L- tyrosine amide of p- aminobenzylalcohol is detected over a 12 hour period.

### Example 226: Enzymatic generation of oxidative dye precursor p-hydroquinone from a pro-dye

A solution of the di-glutarate ester of p-hydroquinone was prepared at a concentration of 100 mM in aqueous 200 mM potassium phosphate buffer, and the pH was adjusted to 7.5 by the addition of 6 M sodium hydroxide solution. The pH-adjusted di-glutarate solution was diluted 1:10 (final concentration of 10 mM) into a 200 mM potassium phosphate buffer solution, pH 7.5, containing a suspension of immobilized glutaryl acylase (BioCatalytics, Inc., Pasadena, CA USA; Product Number 1464213) added to achieve a catalytic activity of 104 units per milliliter. As a negative control, an identical reaction mixture was prepared except that the immobilized glutaryl acylase was replaced with penicillin amidase (BioCatalytics, Inc., Pasadena, CA USA; Product Number 1290959) added to achieve a catalytic activity of 400 units per milliliter. The reaction mixtures were mixed gently and the progress of the reaction was followed by thin layer chromatography (silica gel plates, eluent of methanol:methylene chloride 1:10; visualization by UV light). Complete conversion of the di-glutarate of p-hydroquinone to hydroquinone was achieved within 1 hour. In the case of the control reaction with penicillin amidase, no conversion of the di-glutarate of p-hydroquinone was detected over a 6 hour period.

### Example 227: Alternative enzymatic generation of oxidative dye precursor p-hydroquinone from a pro-dye

A suspension of the mono-phenylacetate ester of p-hydroquinone was prepared by the addition of 4 milligrams per milliliter in aqueous 200 mM potassium phosphate buffer, pH 7.5. Penicillin amidase (BioCatalytics, Inc., Pasadena, CA USA; Product Number 1290959) was added to achieve a final concentration of 200 units per milliliter. As a negative control, an identical reaction mixture was prepared except that the penicillin amidase was replaced with immobilized glutaryl acylase (BioCatalytics, Inc., Pasadena, CA USA; Product Number 1464213) added to achieve a catalytic activity of 104 units per milliliter. The reaction mixtures were mixed gently and the progress of the reaction was followed by thin layer chromatography (silica gel plates, eluent of methanol:methylene chloride 1:10; visualization by UV light). Complete conversion of the mono-phenylacetate of p-hydroquinone to hydroquinone was achieved within 1 hour. In the case of the control reaction with glutaryl acylase, no conversion of the mono-phenylacetate of p-hydroquinone was detected over a 4 hour period.

### Example 228: Enzymatic generation of oxidative dye precursor p-hydroquinone from a pro-dye in the presence of a co-solvent

A suspension of the mono-phenylacetate of p-hydroquinone was prepared by the addition of 4 milligrams per milliliter in aqueous 200 mM potassium phosphate buffer, pH 7.5 containing 20% v/v dimethylsulfoxide. Penicillin amidase (BioCatalytics, Inc., Pasadena, CA USA; Product Number 1290959) was added to achieve a final concentration of 200 units per milliliter. As a negative control, an identical reaction mixture was prepared except that the penicillin amidase was replaced with immobilized glutaryl acylase (BioCatalytics, Inc., Pasadena, CA USA; Product Number 1464213) added to achieve a catalytic activity of 104 units per milliliter. The reaction mixtures were mixed gently and the progress of the reaction was followed by thin layer chromatography (silica gel plates, eluent of methanol:methylene chloride 1:10; visualization by UV light). Complete conversion of the mono-phenylacetate of p-hydroquinone to hydroquinone was achieved within 24 hours. In the case of the control reaction with glutaryl acylase, no conversion of the mono-phenylacetate of p-hydroquinone was detected over a 4 hour period.

### Example 229: Enzymatic generation of oxidative dye precursor p-hydroquinone from a mono-phenylacetate pro-dye in the presence of a co-solvent

A suspension of the mono-phenylacetate of p-hydroquinone was prepared by the addition of 2.6 milligrams per milliliter in aqueous 200 mM potassium phosphate buffer, pH 7.5 containing 20% v/v dimethylsulfoxide. Penicillin amidase (BioCatalytics, Inc., Pasadena, CA USA; Product Number 1290959) was added to achieve a final concentration of 200 units per milliliter. As a negative control, an identical reaction mixture was prepared except that the penicillin amidase was replaced with immobilized glutaryl acylase (BioCatalytics, Inc., Pasadena, CA USA; Product Number 1464213) added to achieve a catalytic activity of 104 units per milliliter. The reaction mixtures were mixed gently and the progress of the reaction was followed by thin layer chromatography (silica gel plates, eluent of methanol:methylene chloride 1:10; visualization by UV light). Complete conversion of the mono-phenylacetate of p-hydroquinone to hydroquinone was achieved within 1 hour. In the case of the control reaction with glutaryl acylase, no conversion of the mono-phenylacetate of p-hydroquinone was detected over a 4 hour period.

### Example 230: Enzymatic generation of direct nitro-dye from a pro-dye with an immobilized enzyme

A solution of 5-[5-amino-6-nitro-2-(pyridinyl)amino]-5-oxopentanoic acid (according to example 1) was prepared at a concentration of 100 mM in aqueous 200 mM potassium phosphate buffer, and the pH was adjusted to 7.5 by the addition of 6 M sodium hydroxide solution. The pH-adjusted glutaramide solution was diluted 1:10 (final concentration of 10 mM) into a 200 mM potassium phosphate buffer solution, pH 7.5, containing a suspension of immobilized glutaryl acylase (BioCatalytics, Inc., Pasadena, CA USA; Product Number 1464213) added to achieve a catalytic activity of 104 units per milliliter. As a negative control, an identical reaction mixture was prepared without the immobilized glutaryl acylase. The reaction mixtures were mixed gently at 37 °C and the progress of the reaction was followed by thin layer chromatography (silica gel plates, eluted with ethylacetate; visualization by UV-light). Complete conversion of the 5-[5-amino-6-nitro-2-(pyridinyl)amino]-5-oxopentanoic acid to 2,5-diamino-6-nitropyridine was achieved within 24 hours. In the case of the control reaction without enzyme, no conversion of the mono-glutaramide of 2,5-diamino-6-nitropyridine was detected over a 24 hour period.

### Example 231: Enzymatic generation of direct nitro-dye from a pro-dye with a non-immobilized enzyme

A solution of 5-[5-amino-6-nitro-2-(pyridinyl)amino]-5-oxopentanoic acid (according to example 1) was prepared at a concentration of 100 mM in aqueous 200 mM potassium phosphate buffer, and the pH was adjusted to 7.5 by the addition of 6 M sodium hydroxide solution. The pH-adjusted glutaramide solution was diluted 1:10 (final concentration of 10 mM) into a 200 mM potassium phosphate buffer solution, pH 7.5, containing the non-immobilized glutaryl acylase (BioCatalytics, Inc., Pasadena, CA USA; Product Number 1479725) added to achieve a catalytic activity of 104 units per milliliter. As a negative control, an identical reaction mixture was prepared without the non-immobilized glutaryl acylase. The reaction mixtures were mixed gently at 37 °C and the progress of the reaction was followed by thin layer chromatography (silica gel plates, eluted with ethylacetate; visualization by UV-light). Complete conversion of the 5-[5-amino-6-nitro-2-(pyridinyl)amino]-5-oxopentanoic acid to 2,5-diamino-6-nitropyridine was achieved within 30 minutes. In the case of the control reaction without enzyme, no conversion of the mono-glutaramide of 2,5-diamino-6-nitropyridine was detected over a 24 hour period.

### Example 232: Enzymatic generation of a direct azo-dye from a pro-dye with an immobilized enzyme

A suspension of 5-[(6-amino-5-(3-pyridinyldiazenyl)-2-pyridinyl)amino]-5-oxopentanoic acid (according to example 2) was prepared at a concentration of 100 mM in aqueous 200 mM potassium phosphate buffer, and the pH was adjusted to 7.5 by the addition of 6 M sodium hydroxide solution. The pH-adjusted glutaramide solution was diluted 1:10 (final concentration of 10 mM) into a 200 mM potassium phosphate buffer solution, pH 7.5, containing a suspension of immobilized glutaryl acylase (BioCatalytics, Inc., Pasadena, CA USA; Product Number 1464213) added to achieve a catalytic activity of 104 units per milliliter. As a negative control, an identical reaction mixture was prepared without the immobilized glutaryl acylase. The reaction mixtures were mixed gently at 37 °C and the progress of the reaction was followed by thin layer chromatography (silica gel plates, eluted with ethylacetate; visualization by UV-light). Complete conversion of the 5-[(6-amino-5-(3-pyridinyldiazenyl)-2-pyridinyl)amino]-5-oxopentanoic acid to 2,6-diamino-3-((pyridin-3-yl)azo)pyridine was achieved within 24 hours. In the case of the control reaction without enzyme, no conversion of the mono-glutaramide of 2,6-diamino-3-((pyridin-3-yl)azo)pyridine was detected over a 24 hour period.

### Example 233: Enzymatic generation of a direct azo-dye from a pro-dye with an immobilized enzyme in the presence of a co-solvent

A suspension of 5-[(6-amino-5-(3-pyridinyldiazenyl)-2-pyridinyl)amino]-5-oxopentanoic acid (according to example 2) was prepared by the addition of 3.28 mg per milliliter in aqueous 200 mM potassium phosphate buffer, pH 7.5 , containing 20 vol% dimethylsulfoxide. Immobilized glutaryl acylase (BioCatalytics, Inc., Pasadena, CA USA; Product Number 1464213) was added to achieve a catalytic activity of 104 units per milliliter. As a negative control, an identical reaction mixture was prepared without the immobilized glutaryl acylase. The reaction mixtures were mixed gently at 37 °C and the progress of the reaction was followed by thin layer chromatography (silica gel plates, eluted with ethylacetate; visualization by UV-light). Complete conversion of the 5-[(6-amino-5-(3-pyridinyldiazenyl)-2-pyridinyl)amino]-5-oxopentanoic acid to 2,6-diamino-3-((pyridin-3-yl)azo)pyridine was achieved within 24 hours. In the case of the control reaction without enzyme, no conversion of the mono-glutaramide of 2,6-diamino-3-((pyridin-3-yl)azo)pyridine was detected over a 24 hour period.

### Example 234: Enzymatic generation of a direct azo-dye from a pro-dye with a non-immobilized enzyme

A suspension of 5-[(6-amino-5-(3-pyridinyldiazenyl)-2-pyridinyl)amino]-5-oxopentanoic acid (according to example 2) was prepared at a concentration of 100 mM in aqueous 200 mM potassium phosphate buffer, and the pH was adjusted to 7.5 by the addition of 6 M sodium hydroxide solution. The pH-adjusted glutaramide solution was diluted 1:10 (final concentration of 10 mM) into a 200 mM potassium phosphate buffer solution, pH 7.5, containing the non-immobilized glutaryl acylase (BioCatalytics, Inc., Pasadena, CA USA; Product Number 1479725) added to achieve a catalytic activity of 104 units per milliliter. As a negative control, an identical reaction mixture was prepared without the non-immobilized glutaryl acylase. The reaction mixtures were mixed gently at 37 °C and the progress of the reaction was followed by thin layer chromatography (silica gel plates, eluted with ethylacetate; visualization by UV light). Complete conversion of the 5-[5-amino-6-nitro-2-(pyridinyl)-amino]-5-oxopentanoic acid to 2,5-diamino-6-nitropyridine was achieved within 30 minutes. In the case of the control reaction without enzyme, no conversion of the mono-glutaramide of 2,5-diamino-6-nitropyridine was detected over a 24 hour period.

### C. Methods and compositions for dyeing

### Example 235: Dyeing using a pro-dye of a nitrobenzene direct dye

### Composition (A):

| | |
|---|---|
| 10 mmoles | glutaramide of 1-amino-3-methyl-4-[(2-hydroxyethyl)- |
| | amino]-6-nitrobenzene (HC Violet No. 1) |
| 100 ml | 1 M potassium phosphate, pH = 7.0 |
| 900 ml | water |

### Composition (B):

| | |
|---|---|
| 1 g | glutaryl acylase (BioCatalytics, Inc., Pasadena, CA USA; |
| | Product number 1479725) |

To fifty milliliters of composition (A) is added 50 milligrams (= approximately 250 units) of composition (B), and the ingredients are mixed to form a dyeing solution. A shock of bleached hair (10 grams) is submerged in the dyeing solution. The dyeing solution containing the submerged hair is agitated slowly by orbital shaking at 37 °C for 30 minutes. At the end of this time, the hair is removed, rinsed under cold running tap water, and dried in air by placement on an absorbent paper towel. The hair is dyed a deep violet color.

### Example 236: Dyeing using a pro-dye of a nitrobenzene direct dye

### Composition (A):

| | |
|---|---|
| 10 mmoles | glutaramide of 1-amino-4-[(2-hydroxyethyl)amino]-2- |
| | nitrobenzene (HC Red No. 7) |
| 100 ml | 1 M potassium phosphate, pH = 7.0 |
| 900 ml | water |

### Composition (B):

| | |
|---|---|
| 1 g | glutaryl acylase (BioCatalytics, Inc., Pasadena, CA USA; |
| | Product number 1479725) |

To fifty milliliters of composition (A) is added 50 milligrams (= approximately 250 units) of composition (B), and the ingredients are mixed to form a dyeing solution. The dyeing is carried out as in Example 235.
The hair is dyed a deep red color.

### Example 237: Dyeing using a pro-dye of a nitro direct dye

### Composition (A):

| | |
|---|---|
| 10 mmoles | glutaramide of 4-amino-2-nitro-diphenylamine (HC Red No. 1) |
| 100 ml | 1 M potassium phosphate, pH = 7.0 |
| 900 ml | water |

### Composition (B):

| | |
|---|---|
| 1 g | glutaryl acylase (BioCatalytics, Inc., Pasadena, CA USA; |
| | Product number 1479725) |

To fifty milliliters of composition (A) is added 50 milligrams (= approximately 250 units) of composition (B), and the ingredients are mixed to form a dyeing solution. The dyeing is carried out as in Example 235.
The hair is dyed a deep red color.

### Example 238: Dyeing using a pro-dye of a nitrobenzene direct dye

### Composition (A):

| | |
|---|---|
| 10 mmoles | glutaramide of 1-amino-2-methyl-6-nitrobenzene |
| 100 ml | 1 M potassium phosphate, pH = 7.0 |
| 900 ml | water |

### Composition (B):

| | |
|---|---|
| 1 g | glutaryl acylase (BioCatalytics, Inc., Pasadena, CA USA; |
| | Product number 1479725) |

To fifty milliliters of composition (A) is added 50 milligrams (= approximately 250 units) of composition (B), and the ingredients are mixed to form a dyeing solution. The dyeing is carried out as in Example 235. The hair is dyed a deep yellow color.

### Example 239: Dyeing using a pro-dye of a quinone direct dye

### Composition (A):

| | |
|---|---|
| 10 mmoles | glutaramide of 1-amino-4-hydroxy-9,10-anthraquinone |
| | (CI60710, Disperse Red 15) |
| 100 ml | 1 M potassium phosphate, pH = 7.0 |
| 900 ml | water |

### Composition (B):

| | |
|---|---|
| 1 g | glutaryl acylase (BioCatalytics, Inc., Pasadena, CA USA; |
| | Product number 1479725) |

To fifty milliliters of composition (A) is added 50 milligrams (= approximately 250 units) of composition (B), and the ingredients are mixed to form a dyeing solution. The dyeing is carried out as in Example 235.
The hair is dyed a deep red color.

### Example 240: Dyeing using a phenylacetamide pro-dye of a nitrobenzene direct dye

### Composition (A):

| | |
|---|---|
| 10 mmoles | phenylacetamide of 1-amino-3-methyl-4-[(2-hydroxyethyl)- |
| | amino]-6-nitrobenzene (HC Violet No. 1) |
| 100 ml | 1 M potassium phosphate, pH = 7.0 |
| 900 ml | water |

### Composition (B):

| | |
|---|---|
| 1 g | penicillin amidase (BioCatalytics, Inc., Pasadena, CA USA; |
| | Product number 1290959) |

To fifty milliliters of composition (A) is added 50 milligrams (= approximately 500-1000 units) of composition (B), and the ingredients are mixed to form a dyeing solution. The dyeing is carried out as in Example 235. The hair is dyed a deep violet color.

### Example 241: Dyeing using a phenylacetamide pro-dye of a nitrobenzene direct dye

### Composition (A):

| | |
|---|---|
| 10 mmoles | phenylacetamide of 1-amino-4-[(2-hydroxyethyl)amino]-2- |
| | nitrobenzene (HC Red No. 7) |
| 100 ml | 1 M potassium phosphate, pH = 7.0 |
| 900 ml | water |

### Composition (B):

| | |
|---|---|
| 1 g | penicillin amidase (BioCatalytics, Inc., Pasadena, CA USA; |
| | Product number 1290959) |

To fifty milliliters of composition (A) is added 50 milligrams (= approximately 500-1000 units) of composition (B), and the ingredients are mixed to form a dyeing solution. The dyeing is carried out as in Example 235.
The hair is dyed a deep red color.

### Example 242: Dyeing using a phenylacetamide pro-dye of a nitro direct dye

### Composition (A):

| | |
|---|---|
| 10 mmoles | phenylacetamide of 4-amino-2-nitro-diphenylamine (HC Red |
| | No. 1) |
| 100 ml | 1 M potassium phosphate, pH = 7.0 |
| 900 ml | water |

### Composition (B):

| | |
|---|---|
| 1 g | penicillin amidase (BioCatalytics, Inc., Pasadena, CA USA; |
| | Product number 1290959) |

To fifty milliliters of composition (A) is added 50 milligrams (= approximately 500-1000 units) of composition (B), and the ingredients are mixed to form a dyeing solution. The dyeing is carried out as in Example 235.
The hair is dyed a deep red color.

### Example 243: Dyeing using a phenylacetamide pro-dye of a nitrobenzene direct dye

### Composition (A):

| | |
|---|---|
| 10 mmoles | phenylacetamide of 1-amino-2-methyl-6-nitrobenzene |
| 100 ml | 1 M potassium phosphate, pH = 7.0 |
| 900 ml | water |

### Composition (B):

| | |
|---|---|
| 1 g | penicillin amidase (BioCatalytics, Inc., Pasadena, CA USA; Product number 1290959) |

To fifty milliliters of composition (A) is added 50 milligrams (= approximately 500-1000 units) of composition (B), and the ingredients are mixed to form a dyeing solution. The dyeing is carried out as in Example 235. The hair is dyed a deep yellow color.

### Example 244: Dyeing using a phenylacetamide pro-dye of a quinone direct dye

### Composition (A):

| | |
|---|---|
| 10 mmoles | phenylacetamide of 1-amino-4-hydroxy-9,10-anthraquinone |
| | (CI60710, Disperse Red 15) |
| 100 ml | 1 M potassium phosphate, pH = 7.0 |
| 900 ml | water |

### Composition (B):

| | |
|---|---|
| 1 g | penicillin amidase (BioCatalytics, Inc., Pasadena, CA USA; |
| | Product number 1290959) |

To fifty milliliters of composition (A) is added 50 milligrams (= approximately 500-1000 units) of composition (B), and the ingredients are mixed to form a dyeing solution. The dyeing is carried out as in Example 235.
The hair is dyed a deep red color.

### Example 245: Dyeing using a leucine amide pro-dye of a quinone direct dye

### Composition (A):

| | |
|---|---|
| 10 mmoles | L-leucinamide of 1-amino-4-hydroxy-9,10-anthraquinone |
| | (CI60710, Disperse Red 15)100 ml |
| 1 M | potassium phosphate, pH = 7.0 |
| 900 ml | water |

### Composition (B):

| | |
|---|---|
| 1 g | L-leucine amidase (BioCatalytics, Inc., Pasadena, CA USA; |
| | Product number 1290959) |

To fifty milliliters of composition (A) is added 50 milligrams (= approximately 500-1000 units) of composition (B), and the ingredients are mixed to form a dyeing solution. The dyeing is carried out as in Example 235.
The hair is dyed a deep red color.

### Example 246: Dyeing using an aspartic acid amide pro-dye of a quinone direct dye

### Composition (A):

| | |
|---|---|
| 10 mmoles | L-aspartic acid β-amide of 1-amino-4-hydroxy-9,10- |
| | anthraquinone (CI60710, Disperse Red 15) |
| 100 ml | 1 M potassium phosphate, pH = 7.0 |
| 900 ml | water |

### Composition (B):

| | |
|---|---|
| 1 g | papain (Sigma Chemical Co., St. Louis, MO USA) |

To fifty milliliters of composition (A) is added 50 milligrams (= approximately 250 units) of composition (B), and the ingredients are mixed to form a dyeing solution. The dyeing is carried out as in Example 235.
The hair is dyed a deep red color.

### Example 247: Dyeing using a phenylacetamide pro-dye of an oxidative dye

### Composition (A):

| | |
|---|---|
| 10 mmoles | phenylacetamide of p-phenylenediamine |
| 100 ml | 1 M potassium phosphate, pH = 7.0 |
| 900 ml | water |

### Composition (B):

| | |
|---|---|
| 10 mmoles | 3-amino-2-chloro-6-methyl-phenol |
| 100 g | laurylethersulfate (28 % aqueous solution) |
| 90 g | ammonia (22 % aqueous solution) |
| 78 g | ethanol |
| 3 g | ascorbic acid |
| ad 1000 g | water |

To fifty milliliters of composition (A) is added 50 milliliters of composition (B), 50 millilters of 6% hydrogen peroxide, and 50 milligrams (approximately 500-1000 units) penicillin amidase (BioCatalytics, Inc., Pasadena, CA USA; Product number 1290959), and the ingredients are mixed to form a dyeing solution. Bleached hair was treated with the aforementioned dyeing solution at 37 °C for 30 minutes. At the end of this time, the hair is rinsed under lukewarm water, and dried. The hair is dyed a deep color.

### Example 248: Dyeing using a glutaramide pro-dye of an oxidative dye

### Composition (A):

| | |
|---|---|
| 10 mmoles | glutaramide of p-phenylenediamine |
| 100 ml | 1 M potassium phosphate, pH = 7.0 |
| 900 ml | water |

### Composition (B):

| | |
|---|---|
| 10 mmoles | 2-methyl-1,3-dihydroxy-benzene |
| 100 g | laurylethersulfate (28 % aqueous solution) |
| 90 g | ammonia (22 % aqueous solution) |
| 78 g | ethanol |
| 3 g | ascorbic acid |
| ad 1000 g | water |

To fifty milliliters of composition (A) is added 10 milliliters of composition (B), 10 millilters of 30% hydrogen peroxide, and 50 milligrams (approximately 250 units) glutaryl acylase (BioCatalytics, Inc., Pasadena, CA USA; Product number 1479725), and the ingredients are mixed to form a dyeing solution. Bleached hair was treated with the aforementioned dyeing solution at 37 °C for 30 minutes. At the end of this time, the hair is rinsed under lukewarm water, and dried. The hair is dyed a deep color.

Unless stated otherwise, all percentages in the present application are percentages by weight.

## Claims

1. Composition for colouring dye-susceptible material, **characterized in that** it contains at least one pro-dye and at least one enzyme capable of reacting, cleaving, or modifying the enzymatically-labile functionality in the pro-dye to form a dye substance or direct dye.

2. Composition according to claim 1, **characterized in that** the pro-dye is selected from the group consisting of amide derivatives of direct dyes containing one or more amine functional groups, amide derivatives of reactive dyes, amide derivatives of oxidative dye precursors containing one or more amine functional groups, ester derivatives of direct dyes containing one or more hydroxy functional groups, ester derivatives of oxidative dye precursors containing one or more hydroxy functional groups, and mixtures thereof.

3. Composition according to claim 1 or 2, **characterized in that** said amide derivative is selected from the group consisting of amides formed from natural or non-naturally occurring amino acids that can be cleaved with a suitable protease, amidase or peptidase; acetamides, propionamides, butyramides, benzamides, phenylacetamides, substituted phenylacetamides, succinamides, glutaramides, adipic acid amides and phthalamides of said direct dyes, said reactive dyes and said oxidative dye precursors.

4. Composition according to claim 1 or 2, **characterized in that** said ester derivative is selected from the group consisting of ester derivatives of amino acids, acetates, propionates, butyrates, benzoates, substituted benzoates, phenylacetates, substituted phenylacetates, succinates, glutarates, adipates, phthalates, palmitates, oleates, stearates, glycerates and gluconates of said direct dyes and said oxidative dye precursors.

5. Composition according to claim 3, **characterized in that** said glutaramide is the glutaramide compound of formula (I) 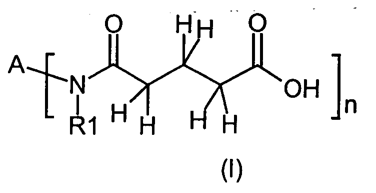 in which
**R1** denotes a hydrogen atom, a C1-C4-alkyl radical or a C1-C4-hydroxyalkyl radical;
**A** denotes the aromatic or heteroaromatic moiety of a mono or diamino dye precursor,
and **n** is 1 or 2.

6. Composition according to anyone of claims 1 to 5, **characterized in that** it additionally contains a coupler substance.

7. Composition according to anyone of claims 1 to 6, **characterized in that** said enzyme capable of reacting, cleaving, or modifying the enzymatically-labile functionality in the pro-dye is selected from the group consisting of esterases, lipases, proteases, peptidases, amidases, acylases and mixtures thereof.

8. Composition according to anyone of claims 1 to 7, **characterized in that** said pro-dye or glutaramide of formula (I) or (la) is contained in an amount of from 0.01 to 25 % percent by weight.

9. Composition for colouring keratin fibres in the form of a 2-component-kit, consisting of a component (A) containing the pro-dye, and a component (B) containing the enzyme capable of reacting, cleaving, or modifying the enzymatically-labile functionality in the pro-dye to form a dye substance or a direct dye.

10. Composition for colouring keratin fibres in the form of a 2-component-kit, consisting of a component (A) containing the pro-dye, the enzyme capable of reacting, cleaving, or modifying the enzymatically-labile functionality in the pro-dye to form a dye substance or a direct dye, and having a pH at which the enzyme is stable but not active, and a component (B) containing a pH-adjuster capable of adjusting the pH of component (A) into the range at which the enzyme is active.
